# EUROPEAN PATENT SPECIFICATION

(11) **EP 2 046 385 B1**
(45) Date of publication and mention of the grant of the patent: **25.03.2015**
(21) Application number: 07796639.8
(22) Date of filing: 02.07.2007
(51) Int. Cl.: A61K 39/395, C07K 16/00, A61K 45/06, C07K 16/44, A61K 39/00

(54) **COMPOSITION FOR MODULATING THE EXPRESSION OF CELL ADHESION MOLECULES**
ZUSAMMENSETZUNG ZUR MODULATION DER EXPRESSION VON ZELLADHÄSIONSMOLEKÜLEN
COMPOSITION SERVANT À MODULER L'EXPRESSION DE MOLÉCULES D'ADHÉRENCE CELLULAIRE

(30) Priority: 03.07.2006 US 818313 P
(43) Date of publication of application: 15.04.2009
(73) Proprietor: Adair, Charles David, Signal Mountain TN 37377-2122 (US)
(72) Inventor: Adair, Charles David, Signal Mountain TN 37377-2122 (US)
(74) Representative: Hiebl, Inge Elisabeth
(86) International application number: PCT/US2007/015348
(87) International publication number: WO 2008/005429

(56) References cited:
- WO-A-2004/011028
- MATSUMORI AKIRA ET AL: "High doses of digitalis increase the myocardial production of proinflammatory cytokines and worsen myocardial injury in viral myocarditis: A possible mechanism of digitalis toxicity" JAPANESE CIRCULATION JOURNAL, vol. 63, no. 12, December 1999 (1999-12), pages 934-940, XP002539466 ISSN: 0047-1828
- YIN ET AL: "Increased endothelial monocyte adhesiveness is related to clinical outcomes in chronic heart failure" INTERNATIONAL JOURNAL OF CARDIOLOGY, ELSEVIER SCIENCE PUBLISHERS, AMSTERDAM, NL, vol. 121, no. 3, 22 December 2006 (2006-12-22), pages 276-283, XP022266738 ISSN: 0167-5273
- WANG Y ET AL: "Digibind attenuates cytokine TNF[alpha]-induced endothelial inflammatory response: Potential benefit role of Digibind in preeclampsia" JOURNAL OF PERINATOLOGY 2009 GB, vol. 29, no. 3, 2009, pages 195-200, XP009120899 ISSN: 0743-8346 1476-5543
- BEHRINGER W. ET AL: 'Percutaneous cardiopulmonary bypass for therapy resistant cardiac arrest from digoxin overdose' RESUSCITATION vol. 37, no. 1, 1998, pages 47 - 50, XP008102389
- LEDINGHAM I.M.: 'Heart Failure in Experimental Refactory Shock' EUROP. J. INTENS. CARE MED. vol. 2, 1976, pages 111 - 117, XP008102391
- ARCHER L.T.: 'Myocardial dysfunction in endotoxin and E. coli-induced shock: pathophysiological mechanisms' CIRC. SHOCK vol. 15, no. 4, 1985, pages 261 - 280, XP008102177
- COALSON ET AL.: 'Effects of digoxin on myocardial ultrastructure in endotoxin shock' SURG. GYNECOL. OBSTET. vol. 135, no. 6, 1972, pages 908 - 912, XP008102181
- VALLES J.B.: 'Use of Digoxin to Improve Heart Function in Sepsis' MEDSCAPE CRITICAL CARE vol. 4, no. 1, 2003, pages 1 - 2, XP008102142
- DART R.C.: 'Antibodies as therapeutic agents: The antivenoms' J. OF NATURAL TOXINS. vol. 04, no. 02, 1995, pages 155 - 163, XP008102179
- MENEZES ET AL.: 'Digoxin antibody decreases natriuresis and diuresis in cerebral hemorrhage' INTENSIVE CARE MED. vol. 29, no. 12, 2003, pages 2291 - 2296, XP008102174
- MENEZES AND DICHTCHEKENIAN: 'Digoxin antibody prevents cerebral hemorrhage-induced hypertension' AMERICAN JOURNAL OF HYPERTENSION vol. 16, no. 12, December 2003, pages 1062 - 1065, XP008102172
- DIGIFAB-TM. DIGOXIN IMMUNE FAB (OVINE). 30 Augustus 2001. page 1-14 XP008102157
- DIGIBIND(R). DIGOXIN IMMUNE FAB (OVINE). September 2003, page 1-8 XP008102173
- DOUZINAS EMMANUEL E ET AL: "The level of hypotension during hemorrhagic shock is a major determinant of the post-resuscitation systemic inflammatory response: an experimental study", BMC PHYSIOLOGY, BIOMED CENTRAL LTD., LONDON, GB, vol. 8, no. 1, 18 July 2008 (2008-07-18), page 15, XP021035766, ISSN: 1472-6793
- NATHAN C ET AL: "Points of control in inflammation", NATURE: INTERNATIONAL WEEKLY JOURNAL OF SCIENCE, NATURE PUBLISHING GROUP, UNITED KINGDOM, vol. 420, 19 December 2002 (2002-12-19), pages 846-851, XP002988556, ISSN: 0028-0836, DOI: 10.1038/NATURE01320

## Description

### BACKGROUND OF THE INVENTION

*1. Field of the Invention.* The present invention relates generally to immunology and medicine and more particularly to pharmaceutical compositions for use in the treatment of chronic and acute disorders, diseases, infections and injuries that are characterized by localized or systemic inflammatory responses and associated with or mediated or modulated by TNF.

*2. Background of the Invention.* Many infectious states, and non-infectious disorders and pathologies have localized or systemic inflammation as an underlying feature. Inflammation is an innate immune response and a necessary defensive reaction to infection, tissue damage and injury. The primary objective of inflammation is to prevent infiltration of infectious agents or localize and eradicate an infection, and to repair the surrounding tissue. An inflammatory response consists of exudative and cellular processes. The exudative process involves the movement of fluid containing proteins such as fibrin and immunoglobulins, dilation of blood vessels upstream and constriction of blood vessels downstream of an infection or injury, and increased capillary permeability in the affected tissue resulting in a net loss of blood plasma into the tissue (edema). The cellular process involves a sequence of adhesion and activation events for individual leukocytes, endothelial and other cell types. Leukocyte and endothelial cell activation result in extravasation of leukocytes from the vasculature into the infected or damaged tissue, where they act as phagocytes to remove bacteria and cellular debris and elicit further inflammatory or immune responses. If the injurious agent persists or if there is a defect in regulation of the inflammatory or immune responses that prevents immune homeostasis within the organism, chronic or systemic inflammation may occur. Chronic or systemic inflammation results in continuing extravasation of leukocytes into tissues and the release by macrophages of toxins (including reactive oxygen species) that damage the organism's tissues.

Systemic inflammatory response syndrome ("SIRS") occurs when inflammation has a deleterious effect on the vascular endothelia and major organ systems. Systemic inflammation is referred to as "sepsis" when caused by bacteria, virus, fungi or parasites. Septic shock occurs if sepsis or SIRS results in hypotension that persists despite adequate fluid resuscitation. Hemorrhage may also lead to symptoms that mirror septic shock, such as tissue ischemia, organ dysfunction and vascular failure. Sepsis is a major cause of morbidity and mortality after trauma, including severe hemorrhage and bums. Each year, more than 750,000 people in the United States will develop severe sepsis, and more than 215,000 will die from the condition.

A patient may be at risk for sepsis or SIRS if the patient is very young (premature neonate) or very old, or has one or more of the following: wounds or injuries or burns; critical illness; severe community-acquired pneumonia; intra-abdominal surgery; meningitis; chronic disease (including diabetes, heart failure, chronic renal failure, and chronic obstructive pulmonary disease); compromised immune status (HIV/AIDS, use of cytotoxic and immunosuppressive agents, such as chemotherapy, radiation therapy and steroids); cellulitis; urinary tract infection; or alcohol or drug addition.

SIRS and sepsis are exaggerated responses to infection and injury. These exaggerated responses disrupt homeostasis through an uncontrolled cascade of inflammation, coagulation, and impaired fibrinolysis, causing systemic vasodilation that leads to decreased organ and tissue perfusion and disseminated intravascular coagulation ("DIC"). Severe hemorrhage, sepsis and SIRS may lead to global tissue hypoxia and tissue damage, leading to shock (hypotension despite adequate fluid resuscitation) and multiple organ dysfunction syndrome ("MODS"), often resulting in death. Anti-infective agents, resuscitation and supportive care do not necessarily prevent microvasculature dysfunction or progressive organ dysfunction, which may continue despite adequate oxygen delivery.

SIRS and sepsis are generally diseases of the microvasculature. Microvascular dysfunction is characterized by decreased perfusion and oxygen availability, leading to microvascular thrombosis and reduction in functional capillary density. Microvascular dysfunction also is characterized by endothelial dysfunction, including reduced vasomotor tone, increased capillary permeability, endothelial apoptosis, and increased expression of adhesion molecules resulting in increased leukocyte-endothelium interactions. Activation of leukocytes and endothelial cells, and interaction among such cells, are requisite to inflammation.

Endothelial cells are involved in the regulation of leukocyte extravasation, cytokine and chemokine production, antigen presentation, protease and extracellular matrix synthesis, vasodilation, vascular permeability and angiogenesis. Being at the origin of the inflammatory cascade, endothelial cells play a critical role in the pathophysiology of numerous conditions, disorders and diseases involving inflammation and the innate and adaptive immune responses. Therefore, endothelial cells are important targets for therapeutic intervention in disorders and diseases that have aspects of inflammatory and/or immune responses.

Leukocyte adhesion and activation occurs through a cascade of events including capture by endothelial cells, rolling along the endothelial cell lining, slow rolling, firm adhesion to the endothelial cell surface, and transmigration into the affected tissue via the intercellular space of adjacent endothelial cells. Rolling occurs through low affinity, reversible binding by a general class of cell adhesion molecules known as selectins, including endothelial selectin ("E-selectin"), platelet selectin ("P-selectin") and leukocyte selectin ("L-selectin"). Endothelial cells express P-selectin and E-selectin. L-selectin is expressed on the leukocyte cell surface and, in addition to facilitating leukocyte-endothelial interactions, also facilitates adhesion between leukocytes, thereby enhancing the effect of an individual leukocyte-endothelial cell interaction.

Firm adhesion between leukocytes and endothelial cells, a high affinity and essentially stationary connection, is facilitated by classes of leukocyte cell adhesion molecules known as integrins and adhesion molecules from the immunoglobulin superfamily. The immunoglobulin superfamily includes, among others, intercellular adhesion molecule ("ICAM" e.g., ICAM-1 and ICAM-2), vascular adhesion molecule-1 ("VCAM"), neural adhesion molecule ("NCAM") and various junctional adhesion molecules ("JAMs", e.g. JAM-A, JAM-B, JAM-C). Although adhesion molecules normally are expressed on cell surfaces, they may also exist as soluble molecules found in the circulation and in extracellular fluids; increased concentrations of soluble adhesion molecules and cell surface expression of certain adhesion molecules have been associated with certain pathologies.

Cytokines play a critical role in limiting and controlling pathogenic infections and regulating inflammation and immune responses. Homeostasis between proinflammatory cytokines and anti-inflammatory cytokines is necessary to maintain appropriate inflammatory and immune responses. Proinflammatory cytokines include certain interferons ("IFN"), tumor necrosis factors ("TNF") and certain interleukins ("IL"). When there is infection, injury and/or cellular damage, TNF is secreted by leukocytes (primarily activated macrophages, activated T-lymphocytes, natural killer cells, mast cells and basophils), fibroblasts, endothelial cells, brain astrocytes and other cells. TNF promotes production of other proinflammatory cytokines including, without limitation, interferons (e.g., interferon gamma or "IFN-γ) and various interleukins (e.g., IL-1β, IL-1αβ, IL-6 and IL-8). Similarly, other proinflammatory cytokines may promote production of TNF.

TNF and other proinflammatory cytokines also activate cells to, among other things, increase cell surface expression of cellular adhesion molecules (receptors and ligands on cell surfaces), and stimulate the release of platelet activating factor, inducing procoagulant activity on vascular endothelial cells and increasing the adherence of leukocytes. The increase in cell adhesion molecules results in increasing leukocyte-endothelial cell interaction, rolling, adhesion and extravasation of neutrophils, monocytes, activated T-helper and T-cytotoxic cells, and memory T and B cells into the infected or damaged tissue. In the tissue, T and B cell activation causes release of antibody and complement. Cellular adhesion molecules that induced by TNF and other proinflammatory cytokines include, among others, VCAM-1, ICAM-1, and E-selectin.

TNF and other proinflammatory cytokines, and their respective receptors, are current targets of therapeutic intervention in infections and inflammatory and immune disorders. A number of anti-cytokine therapies are currently in clinical trials or have been proposed for clinical use in the treatment of sepsis, psoriasis, rheumatoid arthritis, ankylosing spondolytis, Crohn's disease, inflammatory bowel disease and ulcerative colitis. Monoclonal TNF-α antibody, fully human TNF-α antibody, and polyclonal TNF antibody have been suggested as possible treatments for one or more inflammation-related conditions. See U.S. Pat. Nos. 7,012,135, 6,090,382, 6,509,015 and 6,193,969. WO 2004/011028 discloses a method of regulating a preeclamptic/eclamptic patient's sodium/potassium ATPase activity including the administration of digoxin immune Fab (ovine).

Behringer W., et al., "Percutaneous cardiopulmonary bypass for therapy resistant cardiac arrest from digoxin overdose", Resuscitation, vol. 37, no. 1, 1998 pages 47-50, XP008102389 discloses that in cardiac arrest due to digoxin intoxication percutaneous cardiopulmonary bypass may be able to maintain adequate tissue perfusion and sufficient distribution of the antidote to achieve neutralization of the drug. Digoxin inhibits membrane-bound Na⁺, K⁺ -ATPase which leads to increased calcium availability and results in improved cardiac muscle contraction. Immunotherapy with antidigoxin-Fab fragments is the specific treatment for digitalis intoxication.

Dart R.C.: "Antibodies as therapeutic agents: The antivenoms", J. of Natural Toxins, vol. 04, no. 02, 1995, pages 155-163, XP008102179 discloses that polyclonal Fab, e.g. digoxin Fab, to be effective as well as safe for the treatment of septic shock.

Menezes et al.: "Digoxin antibody decreases natriuresis and diuresis in cerebral hemorrhage", Intensive Care Med., vol. 29, no. 12, 2003, pages 2291-2296, XP008102174 discloses that digoxin antibody administration reverses an increase in serum oubain-like compound (OLC) and blood pressure (BP), thus showing an antihypertensive effect.

Menezes and Dichtchekenian: "Digoxin antibody prevents cerebral hermorrhage-induced hypertension", American Journal of Hypertension, vol. 16, no. 12, December 2003, pages 10621065, XP008102172 discloses that brain injury may induce hypertension which may be prevented by digoxin antibodies.

DIGIFAB-TM. Digoxin immune Fab (ovine). 30 August 2001. page 1-14, XP008102172 contains a prescribing information as regards the application of DIGIBIND Digoxin Immune Fab (Ovine).

DIGIBIND(R). Digoxin Immune Fab (ovine). September 2003, page 1-8, XP008102173 contains a prescribing information as regards the application of DIGIBIND Digoxin Immune Fab (Ovine.)

In addition, other substances that have inhibitory or antagonistic effects on the production or action of TNF or other proinflammatory cytokines, and agonists of anti-inflammatory cytokines and their receptors, are also expected to be effective the therapeutic agents for cytokine-mediated pathologies. See, e.g., U.S. Patent Nos. 7,034,031 and 7,018,626. Inhibitors or antagonists of cell adhesion molecules are thought to be suitable targets for intervention because these cell adhesion molecules play a fundamental role in development of the inflammatory and immune response. However, recent evidence indicates that use of antibodies to proinflammatory cytokines or their receptors, may cause an increased risk of serious infection and cancer, perhaps because the inhibition or blocking by the antibody limits the patient's ability to mount appropriate inflammatory and immune responses when needed.

Thus, there is a need for a therapeutic composition to treat conditions mediated by proinflammatory cytokines that modulates or attenuates, but does not completely block or inhibit, the effect of proinflammatory cytokines. It would be particularly useful for the therapeutic composition to modulate leukocyte and/or endothelial cell adhesion molecules (e.g., ICAM-1, VCAM-1, and E-selectin), the regulation of which may be induced by proinflammatory cytokines or other endogenous substances that promote an inflammatory-type response.

The present invention is directed to overcoming one or more of the problems set forth above, including overcoming the lack of a pharmaceutical composition that is effective for use in the treatment of localized or systemic inflammatory responses associated with or mediated or modulated by TNF:
The discussion herein related to inflammatory conditions includes those associated with proinflammatory cytokines or other inflammation inducers, their respective receptors, and those conditions associated with an impairment in the expression or regulation of anti-inflammatory cytokines or their receptors or other substances that may be involved in modulating proinflammatory or anti-inflammatory responses. The discussion herein related to inflammatory-type conditions, disorders and diseases include those related to the increased production and/or release of TNF, including, but not limited to, other proinflammatory cytokines (e.g. IL-1 or IL-6) that are modulated by TNF or that modulate TNF. For example, an IL-1 associated disease state, where IL-1 production or action is enhanced in response to TNF, would be considered a disease state associated with TNF. Similarly, an IL-1 associated condition, wherein IL-1 expression or release is increased and thereby causes an increase in TNF, is also considered a disease state that is mediated or modulated by TNF.

For purposes of the invention disclosed herein, chronic and acute disorders, diseases, infections and injuries that are characterized by localized or systemic inflammatory responses and are therefore deemed to be associated with, or mediated or modulated by TNF include, without limitation: (1) diseases and disorders involving the gastrointestinal tract and associated tissues (such as appendicitis, peptic, gastric and duodenal ulcers, peritonitis, pancreatitis, ulcerative colitis, pseudomembranous colitis, acute and ischemic colitis, diverticulitis, periodontal disease, epiglotitis, achalasia, cholangitis, cholecystitis, celiac disease, hepatitis, cirrhosis, inflammatory bowel disease, Crohn's disease, enteritis, and Whipple's disease); (2) severe hemorrhage and systemic or local inflammatory diseases and conditions (such as hay fever, asthma, allergy, anaphylactic shock, immune complex disease, organ ischemia, reperfusion injury, organ necrosis, hemorrhagic shock, sepsis, septic shock, septicemia, Systemic Inflammatory Response Syndrome (SIRS), Compensatory Anti-Inflammatory Response Syndrome (CARS), multiple organ dysfunction syndrome (MODS), multiple organ failure (MOF), endotoxic shock, cachexia, hyperpyrexia, eosinophilic granuloma, granulomatosis, and sarcoidosis); (3) diseases and conditions involving the urogenital system and associated tissues (such as spontaneous preterm labor, placental abruption, recurrent fetal loss, septic abortion, epididymitis, vaginitis, prostatitis, glomerulonephritis and urethritis); (4) diseases and disorders involving the respiratory system and associated tissues (such as bronchitis, emphysema, rhinitis, cystic fibrosis, pneumonia, pneumonitis, chronic obstructive pulmonary disease (COPD), adult (acute) respiratory distress syndrome (ARDS), pneumoultramicroscopicsilico-volcanoconiosis, alveolitis, bronchiolitis, pharyngitis, pleurisy, and sinusitis); (5) infection, and diseases arising from infection, by various viruses, bacterial, fungi, protozoal and multicellular parasites (such as influenza viruses, respiratory syncytial viruses, Human Immunodeficiency Viruses (HIV), hepatitis viruses, herpes viruses, cytomegalovirus, meningitis and adenovirus, gram positive and gram negative bacteria, tuberculosis, leprosy); (6) dermatological diseases and conditions of the skin (such as psoriasis, allergic and acute dermatitis, actinic keratosis, dermatomyositis, chemical and other burns, sunburn, urticaria, warts, and wheals); (7) diseases and conditions involving the blood, lymphatic and cardiovascular systems and associated tissues (such as tissue or organ ischemia, reperfusion injury, vasculitis, lymph edema, angiitis, endocarditis, arteritis, atherosclerosis, thrombosis and thrombophilia, thrombophlebitis, pericarditis, congestive heart failure, myocarditis, myocardial ischemia, ischemic stroke, periarteritis nodosa, restenosis, chemotherapy related anemia, and rheumatic fever); (8) diseases and conditions involving the central or peripheral nervous system and associated tissues (such as Alzheimer's disease, meningitis, encephalitis, demyelinating diseases, multiple sclerosis, myasthenia gravis, cerebral infarction, cerebral embolism, Guillane-Barre syndrome, neuritis, neuralgia, spinal cord injury, brain injury, paralysis, and uveitis); (9) neuropathic pain and diseases and conditions of the bones, joints, muscles and connective tissues (such as the various arthritides and arthralgias, bone resorption diseases, muscle degeneration, anorexia, cachexia, osteoporosis, osteomyelitis, fasciitis, Paget's disease, gout, periodontal disease, rheumatoid arthritis, osteoarthritis, juvenile chronic arthritis (JCA), scleroderma, ankylosing spondylitis, and synovitis); (10) autoimmune and inflammatory conditions and disorders (such as anti-phospohlipid syndrome, myasthenia gravis, thyroiditis, systemic lupus erythematosus, Goodpasture's syndrome, Behcet's syndrome, allograft rejection, graft-versus-host disease, hyperimmunoglobulinemia-D syndrome (HIDS), TNF-receptor associated periodic syndrome (TRAPS), pancreatic beta-cell destruction, insulin resistance, Type I and Type II diabetes, gestational diabetes mellitus, gestational insulin resistance, Berger's disease, and Reiter's syndrome); and (11) various cancers, tumors and proliferative disorders (such as Hodgkin's disease, multiple myeloma, acute and chronic myelogenous leukemia), metastasis, recurrence of cancers and tumors, and injury or trauma resulting from surgical, chemotherapy and radiation treatments of such cancers, tumors and proliferative disorders.

### BRIEF SUMMARY OF THE INVENTION

Patients with sepsis and SIRS demonstrate a rise in plasma K⁺ and a corresponding decrease in intracellular K⁺, and a rise in intracellular Na⁺ and water. These symptoms of sepsis and SIRS suggest that sodium-potassium ATPase ("Na⁺/K⁺ ATPase" or the "sodium pump") may play a role in inflammatory disorders. Natural sodium pump inhibitors are found in certain plants and toads, including classes of substances known as cardenolides and bufadienolides (commonly known as cardiotonic steroids e.g., ouabain, marinofugenin and digitalis). Certain substances that resemble exogenous cardenolides and bufadienolides are found to naturally occur (being endogenous) in mammals, but the function(s) of these endogenous have yet to be fully elucidated. Cardiac glycodsides are used to treat human cardiac disorders and have a narrow therapeutic range. Therefore, antibodies have been developed to a variety of cardenolides and bufadienolides to counteract life-threatening toxicity. Antibodies to cardenolides and bufadienolides act by preventing these substances from binding to the sodium pump.

Surprisingly, it has been discovered that antibodies directed against cardenolides and bufadienolides are useful in modulating the effects of proinflammatory cytokines, such as tumor necrosis factor. The antibodies of the present invention modulate cytokine induced cell surface expression of ICAM, VCAM, and E-selectin, which are critical inflammation, particularly for leukocyte adhesion to vascular endothelia and leukocyte transmigration from the vasculature into tissue. The antibodies also are effective in reducing the cell adhesion molecule expression in cells that has been activated by TNF. Therefore, such antibodies may be useful in both preventing and treating inflammatory conditions, mediated by TNF.

Since endogenous sodium pump inhibitors are known to cross-react with antibodies to exogenous cardenolides and bufadienolides, antibodies specific for endogenous sodium pump inhibitors ("endogenous factors" or "EFs") are also expected to be suitable for modulating cytokine induced cell adhesion molecule expression and may thereby be useful in preventing and treating cytokine-mediated conditions, and antibodies to EFs are included within the present invention.

Accordingly, the present invention relates to a pharmaceutical composition comprising an anti-digoxin antibody composition (as defined herein) for use in the treatment of chronic and acute disorders, diseases, infections and injuries that are characterized by localized or systemic inflammatory responses and associated with or modulated by TNF.

Cytokines, including TNF, when produced in appropriate levels and properly regulated within an organism, play important roles in the cellular life cycle, cellular response to foreign attack and maintenance of homeostasis. Some studies of therapeutic use of anti-TNF antibodies and anti-TNF receptor antibodies have shown that such therapeutic use results in adverse side effects and increased risks for certain infections and pathologies. Therefore, it will be appreciated that the purpose of this invention is not the complete inhibition of the effects of proinflammatory cytokines (e.g., TNF, IL-6, IL-8, etc.) on cell adhesion molecules, but moderation of the cellular response to proinflammatory cytokines, restoration of balance in cellular response between proinflammatory cytokines and anti-inflammatory (immunosuppressive) cytokines during the innate and adaptive immune responses, and thereby the preventing or treating cytokine mediated infections, disorders, conditions, diseases and pathologies.

### BRIEF DESCRIPTION OF THE DRAWINGS

**Fig. 1** is a flow chart illustrating the steps of a method for treating a patient having a cytokine mediated condition.
**Fig. 2A** is a graph illustrating the expression of intercellular adhesion molecule-1 ("ICAM") by human umbilical vein endothelial cells ("HUVECs") that have been induced by TNF-α.
**Fig. 2B** is a graph illustrating the modulation of TNF-α induced expression of ICAM by HUEVCs that have been treated with digoxin antibody before exposure to TNF-α.
**Fig. 3A** is a graph illustrating the expression of vascular cell adhesion molecule-1 ("VCAM") by HUVECs induced by TNF-α.
**Fig. 3B** is a graph illustrating the modulation of TNF-α induced expression of VCAM by HUVECs that have been treated with digoxin antibody before exposure to TNF- α..
**Fig. 4A** is a graph illustrating the expression of vascular cell adhesion molecule E-selectin by HUVECs induced by TNF- α.
**Fig. 4B** is a graph illustrating the modulation of TNF- α induced expression of E-selectin by HUVECs that have been treated with digoxin antibody before exposure to TNF- α.
**Fig. 5A** is a graph illustrating ICAM expression induced by TNF- α and the attenuation of that expression when HUVECs are subsequently treated with digoxin antibody.
**Fig. 5B** is a graph illustrating VCAM expression induced by TNF- α and the attenuation of that expression when HUVECs are subsequently treated with digoxin antibody.
**Fig. 6** is a graph illustrating E-selectin expression induced by TNF- α and the attenuation of that expression when HUVECs are subsequently treated with digoxin antibody.
**Fig. 7A** is an example of the aglycone structure of a bufadienolide.
**Fig. 7B** is an example of the aglycone structure of a cardenolide.

### DETAILED DESCRIPTION OF THE INVENTION

It is noted that as used herein and in the appended claims, the singular forms "a", "and", and "the" include plural referents unless the context clearly dictates otherwise. Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

**I. DEFINITIONS AND ABBREVIATIONS.** The following terms as used herein shall have the definitions set forth below. When such terms are used in the context as defined below, then such terms may be used in the abbreviated form set forth adjacent to such term.

***"patient"*** means humans and other animals, and is intended to encompass any animal or human subject that may benefit from treatment with the compositions and methods of the present invention.

***"sodium pump" or "sodium*/*potassium ATPase" or "Na*⁺/*K*⁺ *ATPase"*** means sodium-potassium adenosine triphosphatase, a transmembrane protein that utilizes energy generated from adenosine triphosphate ("ATP") hydrolysis (ATP → ADP + PO₄) to transport sodium and potassium ions across cell membranes in opposite directions against their chemical and electrical gradients. The sodium pump is the primary transporter responsible for maintaining the electrochemical gradient of Na⁺ across cell membranes and is important in regulating cell volume, cytoplasmic pH, Na⁺- dependent glucose and amino acid transport, and regulating Ca²⁺ levels through the Na⁺/H⁺ and Na⁺/Ca²⁺ ion exchange pumps.

***"endogenous factors" or "EFs"*** means those factors produced in an animal that cross-react immunologically with or that otherwise bind to antibodies specifically directed against at least one epitope of a cardenolide or a bufadienolide, or that are capable of binding to, inhibiting or attenuating the function of Na⁺/K⁺ ATPase. Sometimes herein, EFs may also referred to as digoxin-like substances ("DLS"), digoxin-like immunoreactive substances or factors ("DLIS" or "DLIF"), or endogenous digoxin-like factors ("EDLF").

***"cardenolides (and* / *or) bufadienolides" or "cardiac glycosides"*** means sodium pump inhibitors exogenous to patients. Cardenolides and bufadienolides, and are commonly referred to as cardiotonic steroids. Cardenolides and/or bufadienolides includes the aglycones moieties thereof, including, without limitation, digitalis, gitoxigenin, digoxigenin, digoxin, digitoxigenin, digitoxin, dihydrodigoxin, strophanthins, convallatoxin, cymarine, acetylstrophanthidin, strophanthidin, ouabagenin, ouabain, dihydrooubain, neriifolin, proscillaridin, proscillaridin A, cinobufagen, cinobufatolin, marinobufagenin, norbufalin, bufanolide, bufalin and similar compounds, and their respective isomers, inotropes, congeners, variants, derivatives, equivalents, precursors and metabolites, and synthetic or engineered versions or constructs of any of the foregoing.

***"endothelial activation"*** means a change in phenotype or function of an endothelial cell in response to stimuli from the environment including, without limitation, the expression of molecules that mediate adhesion and/or signaling of leukocytes. Stimuli known to induce activation-dependent functional alterations in human endothelial cells include humoral agonists that interact with cell surface receptors including, but not limited to, cytokines and pleotropic signaling factors such as thrombin, bacterial endotoxin or lipopolysaccharide ("LPS") and other microbial products, hemodynamic perturbations, oxidants, and radiation. Endothelial activation can be a regulated event in homeostatic physiologic vascular responses, or it can be a dysregulated, or unregulated, response in pathologic conditions.

***"systemic inflammatory response syndrome" or "SIRS"*** means a complex of symptoms that result from systemic inflammation, regardless of the cause. Clinical indicators of SIRS includes the presence of more than one of the following: temperature > 100.4°F or < 96.8°F (> 38°C or < 36°C); heart rate > 90 beats/min; tachypnea (a respiratory rate > 20 breaths/min.) or hyperventilation (a PaCO₂ < 32 mm Hg); and white blood cell count > 12,000 cells/mm³ or < 4,000 cells/mm³ or the presence of > 10% immature neutrophils. A systemic inflammatory response leading to a diagnosis of SIRS may be related to both infection and to numerous non-infective etiologies including, without limitation, hemorrhage, burns, pancreatitis, trauma, heat stroke, and neoplasia.

***"sepsis"*** means SIRS resulting from infection (bacterial, viral, fungal, or parasitic), or as otherwise defined from time to time by the American College of Chest Physicians/Society of Critical Care Medicine.

***"severe sepsis"*** means sepsis further accompanied by organ hypoperfusion made evident by at least one sign of organ dysfunction such as hypoxemia, oliguria, metabolic acidosis, or altered cerebral (neurological) function.

**"*septic shock***" means severe sepsis, further accompanied by hypotension, made evident by a systolic blood pressure <90 mm Hg, or the requirement for pharmaceutical intervention to maintain blood pressure.

***"multiple organ dysfunction syndrome" ("MODS")*** means the presence of altered function of two or more organs in an acutely ill patient, such that homeostasis cannot be maintained without intervention.

***"cytokine"*** means any secreted hormone, peptide, polypeptide, protein or other substance that affects a function of a cell and/or that modulates interactions between cells involved in the immune, inflammatory or hematopoietic response. A cytokine includes, but is not limited to, monokines, lymphokines and chemokines, regardless of the cellular source of the cytokine. For instance, a monokine is generally referred to as being produced and secreted by a mononuclear cell, such as a macrophage and/or monocyte. However, many other cells also produce monokines, such as natural killer cells, fibroblasts, basophils, neutrophils, endothelial cells, brain astrocytes, bone marrow stromal cells, epidermal keratinocytes and B-lymphocytes. Examples of cytokines include, but are not limited to, interferons (including IFN-α, IFN-β, IFN-y, etc.), interleukins (including IL-1, IL-2, IL-4, IL-6, IL-8, IL-10, IL-12, IL-17, IL-18, IL-19, etc.), tumor necrosis factors ("TNF", including TNF-α and TNF-β, etc.), granulocyte-macrophage colony stimulating factor ("GM-CSF"), neutrophil-activating protein-1 ("NAP-1"), neutrophil-activating protein-2 ("NAP-2"), GRO-α, GRO-β, GRO-γ, ENA-78, GCP-2, IP-10, MIG, PF4, RANTES, MIP-1α, MIP-2β, monocyte chemotactic proteins (MCP-1, MCP-2, MCP-3, etc.), eotaxin, and including non-immunological cytokines such as C reactive protein, activated protein C, erythropoietin and thrombopoietin. Immunological cytokines may be categorized into those that promote the proliferation and functioning of helper T-cells type 1 ("T_{H}1-type") that generally are proinflammatory (e.g. IL-1, IFN-γ etc.), and helper T-cells type 2 ("T_{H}2-type") that generally are anti-inflammatory (e.g., IL-4, IL-10, IL-13, TGF-β etc.). Generally, cytokines belonging to one of these subsets tend to inhibit the effects of cytokines belonging to the other subset, modulating each other and creating the tendency toward a balanced inflammatory and/or immune response. The failure to maintain balance or the ability to modulate the inflammatory and immune response may play a significant role in pathogenesis of inflammatory and immune conditions and diseases. For purposes of the invention, a proinflammatory cytokine is deemed to be any molecule, hormone, peptide, polypeptide, protein or other substance, or composition of one or more substances, that directly or indirectly affects leukocyte or endothelial cell activation.

"***modulate***", ***"modulating" or "modulation"*** means, without limitation, with respect to a gene, ribonucleic acid, peptide, polypeptide, protein, hormone or other substance, to affect, regulate, adjust, temper, reduce or inhibit the expression, transcription, translation, modification, cleavage, degradation, storage, translocation, recycling, release, avidity, affinity or activity, or its soluble or bound form, or presence within the cell, cell membrane, cell surface, extracellular fluid, matrix, serum or plasma.

***"elevated level(s)"*** means a higher level of the substance in a tissue or fluid of a patient having an abnormal condition, physiological state, disorder, disease or pathology, as compared to a similar or corresponding tissue or fluid from a person who does not have such a condition, physiological state, disorder, disease or pathology (e.g, containing a basal level of the substance or a level of such substance associated with homeostasis).

***"TNF",*** unless specifically delineated otherwise, means tumor necrosis factor alpha (TNF-α, also known as cachectin), or tumor necrosis factor beta (TNF-β, also known as lymphotoxin), or both.

***"IFN"*** means an interferon. The human type I interferons consist of 13 different alpha isoforms (subtypes with slightly different specificities) - IFN-α (1, 2, 4, 5, 6, 7, 8, 10, 13, 14, 16, 17, 21), and single beta ("IFN-β1"), omega, epsilon and kappa isoforms. The type II interferons consist of IFN gamma ("IFN-γ"). Type III interferon consists of IFN-lambda.

***"cytokine mediated condition"*** means any and all infections, physiological states, conditions, syndromes, disorders, diseases and pathologies in which TNF acts as a proinflammatory agent, whether directly or indirectly, and whether resulting from increased expression (transcription and/or translation), alteration in modification, storage, translocation, release, degradation or recycling of TNF or by any other means, including, without limitation by modulation of any anti-inflammatory cytokine or its receptors or metabolites, or by action of any cytokine (or its receptors or metabolites) that modulates TNF, or whether by TNF causing another cytokine to be translocated or released or otherwise affecting the expression (transcription and/or translation), alteration in modification, translocation, release, degradation or recycling of such other cytokine (or its respective receptors or metabolites), or whether by a decrease in the expression (transcription and/or translation), or alteration in modification, translocation, release, degradation or recycling of a cytokine or a cytokine receptor that regulates, modulates or inhibits (wholly or partially) the effects of TNF. For example, without limitation, a condition in which IL-1 is a component, and whose production or action is exacerbated or secreted in response to TNF would, therefore, be considered a cytokine mediated condition. Similarly, a condition associated with IL-1 which causes the secretion of TNF would also be considered a cytokine mediated condition. "Cytokine mediated condition" includes, but is not limited to, the following: (1) diseases and disorders involving the gastrointestinal tract and associated tissues (such as appendicitis, peptic, gastric and duodenal ulcers, peritonitis, pancreatitis, ulcerative colitis, pseudomembranous colitis, acute and ischemic colitis, diverticulitis, periodontal disease, epiglotitis, achalasia, cholangitis, cholecystitis, celiac disease, hepatitis, cirrhosis, inflammatory bowel disease, Crohn's disease, enteritis, and Whipple's disease); (2) severe hemorrhage and systemic or local inflammatory diseases and conditions (such as asthma, allergy, anaphylactic shock, immune complex disease, organ ischemia, reperfusion injury, organ necrosis, hay fever, hemorrhagic shock, sepsis, septic shock, septicemia, neonatal sepsis, Systemic Inflammatory Response Syndrome (SIRS), Compensatory Anti-Inflammatory Response Syndrome (CARS), multiple organ dysfunction syndrome (MODS), multiple organ failure (MOF), endotoxic shock, cachexia, hyperpyrexia, eosinophilic granuloma, granulomatosis, and sarcoidosis); (3) diseases and conditions involving the urogenital system and associated tissues (such as spontaneous preterm labor, placental abruption, recurrent fetal loss, septic abortion, epididymitis, vaginitis, prostatitis, glomerulonephritis and urethritis); (4) diseases and disorders involving the respiratory system and associated tissues (such as bronchitis, emphysema, rhinitis, cystic fibrosis, pneumonia, pneumonitis, chronic obstructive pulmonary disease (COPD), adult (acute) respiratory distress syndrome (ARDS), pneumoultramicroscopicsilico-volcanoconiosis, alveolitis, bronchiolitis, pharyngitis, pleurisy, and sinusitis); (5) infection and diseases arising from infection by various viruses, bacteria, fungi, protozoal and multicellular parasites (such as influenza viruses, respiratory syncytial viruses, Human Immunodeficiency Viruses (HIV), hepatitis viruses, herpes viruses, cytomegalovirus, meningitis and adenovirus, gram positive and gram negative, tuberculosis, leprosy); (6) dermatological diseases and conditions of the skin (such as psoriasis, allergic and acute dermatitis, actinic keratosis, dermatomyositis, chemical and other burns, sunburn, urticaria warts, and wheals); (7) diseases and conditions involving the blood, lymphatic and cardiovascular systems and associated tissues (such as tissue or organ ischemia, reperfusion injury, lymph edema, vasculitis, angiitis, endocarditis, arteritis, peripheral vascular disease, atherosclerosis, thrombosis and thrombophilia, thrombophlebitis, pericarditis, congestive heart failure, myocarditis, myocardial ischemia or infarction, ischemic stroke, periarteritis nodosa, restenosis, chemotherapy related anemia, and rheumatic fever); (8) diseases and conditions involving the central or peripheral nervous system and associated tissues (such as Alzheimer's disease, meningitis, encephalitis, demyelinating diseases, multiple sclerosis, myasthenia gravis, cerebral infarction, cerebral embolism, Guillane-Barre syndrome, neuritis, neuralgia, spinal cord injury, brain injury, paralysis, and uveitis); (9) neuropathic pain and diseases and conditions of the bones, joints, muscles and connective tissues (such as the various arthritides and arthralgias, bone resorption diseases, muscle degeneration, anorexia, cachexia, osteoporosis, osteomyelitis, fasciitis, Paget's disease, gout, periodontal disease, rheumatoid arthritis, osteoarthritis, juvenile chronic arthritis (JCA), scleroderma, ankylosing spondylitis, and synovitis); (10) other autoimmune and inflammatory conditions and disorders (such as anti-phospohlipid syndrome, myasthenia gravis, thyroiditis, systemic lupus erythematosus, Goodpasture's syndrome, Behcet's syndrome, allograft rejection, graft-versus-host disease, hyperimmunoglobulinemia-D syndrome (HIDS), TNF-receptor associated periodic syndrome (TRAPS), pancreatic beta-cell destruction, insulin resistance, Type I and Type II diabetes, gestational diabetes mellitus, gestational insulin resistance, Berger's disease, and Reiter's syndrome); and (11) various cancers, tumors and proliferative disorders (such as Hodgkin's disease, multiple myeloma, acute and chronic myelogenous leukemia), metastasis, recurrence of cancers and tumors, and injury or trauma resulting from surgical, chemotherapy and radiation treatments of such cancers, tumors and proliferative disorders.

"***therapeutically effective***" means effective for treating a physiological state, condition, disorder, syndrome, infection, disease or pathology, or any symptom thereof. As used herein, "treating" includes, without limitation, preventing (prophylactic), reducing the severity of, controlling, limiting the effects of, delaying the onset of, preventing advancement of, causing regression of, alleviating or ameliorating, one or more of the causes, effects, or physiological or clinical indications of a physiological state, condition, disorder, syndrome, infection, disease or pathology. Preferably, a therapeutically effective composition does not cause any unacceptable or significant adverse effect in the patient, including the worsening of any condition or symptom or cause any complication to such an extent that the risks of the adverse effect outweigh the benefits to be derived from the treatment. A therapeutically effective composition may improve or stabilize one or more medical parameters that, in the absence of the composition, might otherwise worsen or develop into a symptom or complication of an infection, condition, disorder, syndrome, disease or pathology. Within the scope of sound medical judgment, the required dosage of a pharmaceutically active agent (ingredient) or of the pharmaceutical composition containing that active ingredient will vary with the severity of the condition being treated, the duration of the treatment, the nature of adjunct treatment, the gender, weight, age and physical condition (health or illness) of the patient, the specific active ingredient employed, and like considerations discussed more fully herein. In arriving at the therapeutically effective amount for a particular pharmaceutical composition or agent, these risks must be taken into consideration, as well as the fact that the compositions described herein provide pharmaceutical activity at lower dosage levels. When used in connection with a composition of the present invention, "therapeutically effective" includes an amount of digoxin antibody that is effective for modulating or attenuating one or more effects of TNF in one or more cell types or tissues and, thus, producing the desired therapeutic effect on a cytokine mediated condition. In particular, a therapeutically effective amount of digoxin antibody will have the affect of modulating or attenuating endothelial activation by TNF, and most preferably will modulate or attenuate the effect of TNF on endothelial cell surface expression of ICAM, VCAM, P-selectin or E-selectin.

***"another therapeutic agent" or "other therapeutic agent***,*"*means any substance or therapeutic agent (other than the compositions, agents and active ingredients of the present invention) that has been used, is currently used, or is known to be useful or that may be developed in the future for use in connection with a cytokine mediated condition. For example, agents used to treat cytokine mediated conditions include, without limitation: antibodies specific for cytokines or cytokine receptors (e.g., infliximib, enteracept, adalimumab); non-steroidal anti-inflammatory agents; corticosteroids; antibodies to cell adhesion molecules (e.g., antibodies specifically directed against an epitope of ICAM, VCAM, E-selectin, P-selectin, integrins, cadherins and the like); activated protein C; T_{H}-2 type cytokine agonists; T_{H}-1 type cytokine antagonists; and antibiotics suitable for one or more bacterial infections known or believed to be present in the patient. Such other therapeutic agents may be administered, by a means or method and in an amount and administered for a duration commonly used for such agent, contemporaneously or sequentially with a compound of the present invention.

***"corticosteroid"*** means any naturally occurring or synthetic (wholly or partially) steroid hormone which can be derived from cholesterol and is characterized by a hydrogenated cyclopentanoperhydrophenanthrene ring system, whether naturally occurring or synthetic. Corticosteroids may have glucocorticoid and/or mineralocorticoid activity. Corticosteroids include, without limitation, dexamethasone, betamethasone, triamcinolone, triamcinolone acetonide, triamcinolone diacetate, triamcinolone hexacetonide, beclomethasone, dipropionate, beclomethasone dipropionate monohydrate, flumethasone pivalate, diflorasone diacetate, fluocinolone acetonide, fluorometholone, fluorometholone acetate, clobetasol propionate, desoximethasone, fluoxymesterone, fluprednisolone, hydrocortisone, hydrocortisone acetate, hydrocortisone butyrate, hydrocortisone sodium phosphate, hydrocortisone sodium succinate, hydrocortisone cypionate, hydrocortisone probutate, hydrocortisone valerate, cortisone acetate, paramethasone acetate, methylprednisolone, methylprednisolone acetate, methylprednisolone sodium succinate, prednisolone, prednisolone acetate, prednisolone sodium phosphate, prednisolone tebutate, clocortolone pivalate, dexamethasone 21-acetate, betamethasone 17-valerate, isoflupredone, 9-fluorocortisone, 6-hydroxydexamethasone, dichlorisone, meclorisone, flupredidene, doxibetasol, halopredone, halometasone, clobetasone, diflucortolone, isoflupredone acetate, fluorohydroxyandrostenedione, flumethasone, diflorasone, fluocinolone, clobetasol, cortisone, paramethasone, clocortolone, prednisolone 21-hemisuccinate free acid, prednisolone metasulphobenzoate, and triamcinolone acetonide 21-palmitate.

***"composition"*** as, for instance, in a pharmaceutical composition or therapeutically effective composition, means a product comprising the active ingredient(s) and the inert and non-active ingredient(s) (including pharmaceutically acceptable carriers and excipients), as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by mixing a digoxin antibody of the present invention, additional active ingredient(s), and/or pharmaceutically acceptable carriers or excipients.

**"*pharmaceutically acceptable"*** means that the pharmaceutically active ingredient and other ingredients used in the pharmaceutical compositions of the inventions described herein are suitable for use in contact with the cells or tissues of humans and other animals without undue toxicity, irritation, allergic response, and the like, commensurate with a reasonable benefit/risk ratio (i.e., in light of known risks for a pharmaceutical, the benefit outweighs such risks).

***"carrier"*** or "***excipient***" means a solid, semi-solid, liquid, aerosol or gaseous filler, diluent or encapsulating substance. These materials are well known to those skilled in the pharmaceutical arts. Some examples of the substances which can serve as pharmaceutical carriers are: sugars (in solid or aqueous form), such as lactose, glucose, and sucrose; starches, such as corn starch and potato starch; cellulose and its derivatives, such as sodium carboxymethyl cellulose, ethyl cellulose, and cellulose acetate; powdered tragacanth; malt; gelatin; talc; stearic acid; magnesium stearate; calcium sulfate; vegetable oils, such as peanut oil, cottonseed oil, sesame oil, olive oil, corn oil and oil of theobroma; polyols, such as propylene glycol, glycerin, sorbitol, mannitol, and polyethylene glycol; agar; alginic acid; pyrogen-free water and Sterile Water for Injection; isotonic saline; and phosphate buffer solutions, as well as other non-toxic compatible substances used in pharmaceutical formulations. They may comprise liposomes or drug carriers made from lipids or polymeric particles, including biodegradable polymers, or targeted delivery applications, e.g., coupling to antibodies. Wetting agents and lubricants, such as sodium lauryl sulfate, as well as coloring agents, flavoring agents, tableting agents, and preservatives, can also be present. Suitable carriers and excipients are described in the most recent edition of A. Gennaro, Remington The Science and Practice of Pharmacy (ed. 20th, Lippincot & Williams 2000), a standard reference text in the pharmaceutical arts. Carriers and excipients particularly suitable for the present invention are further discussed below. Formulation of the active ingredients and carriers and excipients into pharmaceutical compositions may be performed using any one or more of the methods known in the art.

***"antigen"*** means a substance or a portion of a substance capable of being bound by an antibody and which also may be capable of inducing an animal to produce an antibody that selectively binds to an epitope of that antigen. An antigen may have one or more than one epitope. "Epitope" means the specific antigenic structure recognized by a binding domain. Epitopes usually consist of chemically active surface groupings of molecules such as amino acids or sugar side chains, and have specific three dimensional structural characteristics as well as specific charge characteristics. As used herein "antigen" may also mean any ligand which is capably of being bound (whether specifically or non-specifically) by an antibody, whether or not such ligand would elicit an immune response in a patient.

***"antibody" or "antibodies"*** means an immunoglobulin, peptide, polypeptide, protein or other molecule, whether natural or partly or wholly synthetically engineered or constructed, having a CDR or a binding domain or a structure that is substantially homologous to a CDR or binding domain. Examples of antibodies are the immunoglobulin isotypes and their isotypic subclasses; CDRs, binding fragments and diabodies (as defined below). The term also includes derivatives, functional equivalents and homologies of antibodies, including any peptide comprising a CDR or binding domain, whether natural or wholly or partially synthetic or engineered, and whether polyclonal, monoclonal, humanized, chimeric or fully human. Various methods of producing such antibodies are described in US Patent No. 5,225, 539, US Patent No. 5, 693,761, US Patent No. 5,869,619, US Patent No. 5821,337, US Patent No. 5859,204, US Patent No. 6,946,546, US Patent No. 6,939,543, US Patent No. 6,926,896, US Patent No.6,924,125, US Patent No.6,893,624 and US Patent No. 6,881,557. The terms Fv, Fc, Fd, Fab, Fab' or F(ab')₂ are used with their standard meanings. See, e.g., Harlow et al., Antibodies a Laboratory Manual, Cold Spring Harbor Laboratory (1988).

***"engineered antibody"*** means a type of altered antibody in which a portion of the light and/or heavy chain variable domains of a selected acceptor antibody are replaced by analogous parts from one or more donor antibodies which have specificity for the selected epitope (e.g., without limitation, a chimeric, fully human, or humanized antibody, as opposed to an antibody fragment). For example, such molecules may include antibodies characterized by a humanized heavy chain associated with an unmodified light chain (or chimeric light chain), or vice versa. Engineered antibodies may also be characterized by alteration of the nucleic acid sequences encoding the acceptor antibody light and/or heavy variable domain framework regions in order to retain donor antibody binding specificity. These antibodies can comprise replacement of one or more CDRs (preferably all) from the acceptor antibody with CDRs from a donor antibody.

***"chimeric antibody"*** means a type of engineered antibody which contains a naturally occurring variable region (or portion thereof) derived from a donor antibody in association with a constant region derived from an acceptor antibody.

***"humanized antibody"*** means a type of engineered antibody having its CDRs derived from a non-human donor immunoglobulin, the remaining immunoglobulin-derived parts of the molecule being derived from one or more human immunoglobulins. In addition, framework support residues may be altered to preserve binding affinity. See, e.g., Queen et al., Proc. Natl. Acad. Sci. USA, 86:10029-10032 (1989).

***"diabodies"*** means multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form a binding domain. Binding domains are formed by the association of the first domain of one polypeptide within the multimer with the second domain of another polypeptide within the multimer (e.g., U.S. Patent App. Pub. 20050214860).

***"complementarity determining region"** or **"CDRs"*** means the complementarity determining region amino acid sequences of an antibody that are the hypervariable regions of immunoglobulin heavy and/or light chains. See, e.g., Kabat et al., Sequences of Proteins of Immunological Interest (4th Ed., U.S. Department of Health and Human Services, National Institutes of Health, 1987). There are three heavy chain and three light chain CDRs or CDR regions in the variable portion of a naturally occurring immunoglobulin. Thus, CDRs as used herein for a naturally occurring (non-engineered antibody) refers to all three heavy chain CDRs, or all three light chain CDRs or both all heavy and all light chain CDRs, if appropriate. CDRs provide the majority of contact residues for the binding of the antibody to the antigen or epitope.

***"binding fragment" or "binding fragments"*** means a fragment or fragments of whole antibodies (containing Fc and Fv regions) and naturally occurring or synthetically constructed molecules that have all or a portion of a binding domain, whether specific or non-specific, for a ligand or an antigen or an epitope of an antigen.

***"binding domain**"* means the part of an antibody, or of any other naturally occurring, synthetic, engineered antibody or constructed molecule, including, without limitation, a calycin protein, that binds to and/or is complementary to a part of or all of a ligand or an antigen or an epitope of an antigen.

***"digoxin antibody" or "digoxin antibodies"*** means an antibody or binding fragment, whether naturally occurring or wholly or partially synthetic or engineered, that reacts immunologically with, or otherwise binds to (either specifically or non-specifically), or contains a binding domain or CDR for, digitalis, digoxin, digitoxin, digitoxigenin, digoxigenin, gitoxingenin, or any endogenous digoxin-like factor (such as described in U.S. Patent App. Pub. 20050123999), or any epitope of any one ore more of the foregoing. Digoxin antibodies include antibodies specifically directed against any cardenolide or bufadienolide that has the ability to bind at least one epitope of digitalis, digoxin, digitoxin, digitoxigenin, digoxigenin, gitoxingenin, or any endogenous digoxin-like factor. For example, and without limitation, digoxin antibodies include antibodies specific to digoxin, ouabain, bufalin and marinobufagenin, or to a conjugate of any one of the foregoing. Digoxin antibodies also include calycin proteins, whether naturally occurring or wholly or partially synthetic or engineered, that are capable of binding digitalis, digoxin, digitoxin, digitoxigenin, digoxigenin, or gitoxingenin, or an epitope thereof. The calycin superfamily of proteins is characterized by structural motifs formed by anti-parallel, beta-sheets in a manner similar to the CDR region of immunoglobulins. Lipocalins, fatty acid-binding proteins ("FABPs") and avidins are members of the calycin superfamily of proteins. Calycins are relatively small secreted proteins that are believed to be involved in the binding and transport of hydrophobic molecules. The specificity of binding is determined by the conformation and constituent side-chains of the pocket created by folding of the protein. *In vitro,* many lipocalins can bind with high affinity to a range of hydrophobic molecules not normally encountered in nature. This may represent an inherent ability of the proteins to bind molecules having particular biochemical and structural properties. A calycin can be functionally divided into a "binding domain" and a "targeting domain." The "binding domain" functions to interact with ligands, while the "targeting domain" functions to provide specificity in transporting the bound ligand to a defined site. Known lipocalins include: retinol-binding protein; purpurin; retinoic acid-binding protein; alpha_{2µ} -globin; major urinary protein; bilin-binding protein; alpha-crustacyanin; pregnancy protein 14; beta.-lactoglobin; neutrophil lipocalin and choroid plexus protein; odorant-binding protein; von Ebner's gland protein; probasin; and aphrodisin. Lipocalins appear to have a regulatory influence on the inflammatory cascade and protect against excessive tissue damage. See, Fowler D, FEBS Letters 354:7-11 (1994); Fowler D, J. Molec. Recognition 8:185-195 (1995); Fowler D, Biochem. J. 318:1-14 (1996). Calycin proteins may be engineered to have a prescribed ligand specificity, as described in Schlehuber S and Skerra A, Biophysical Chemistry 96:213-228 (2002), U.S. Patent Application 20050106660 and PCT Applications WO 99/16873, WO 00/75308. As described in the foregoing PCT applications, the bilin-binding protein lipocalin has been specifically engineered to bind digoxigenin and therefore is included within the definition of "digoxin antibody."

***"digoxin binding capacity"*** means the amount of digoxin that is capable of being bound by a given amount of digoxin antibody composition. For example, a single vial of DIGIBIND^{®} contains 38 mg total digoxin antibody and is capable of binding approximately 0.5 mg digoxin, *i.e.,* one vial has 0.5 mg digoxin binding capacity or 0.013 mg digoxin bound per mg of antibody (0.5mg digoxin ÷ 38 mg total antibody). Similarly, a vial of the current formulation of DIGIFAB™ contains 40 mg total digoxin antibody and one vial has 0.5 mg digoxin binding capacity, or 0.0125 mg digoxin bound per mg of antibody (0.5 mg ÷ 40 mg total antibody). The digoxin binding capacity of antibodies that are not specific for digoxin, but nevertheless react or bind with digoxin, may be determined as described below.

***"neutralizing** dose"* when used in connection with "digoxin antibody" or "digoxin antibodies" for treating a cytokine mediated condition, means a digoxin antibody composition having an amount of digoxin binding capacity that would be administered to neutralize a specified amount of TNF in a patient, as if TNF were equivalent to digoxin and if comparable digoxin concentrations were deemed to be poisonous or toxic so as to warrant treatment with digoxin antibodies. For example, for a 100 kg patient suffering from severe sepsis and exhibiting a circulating TNF concentration of 400 pg/ml (0.4 ng/ml), then a neutralizing dose of DIGIBIND^{®} (38 mg antibody; 0.5 mg digoxin binding capacity per vial) according to the product insert would be determined as follows: (0.4 ng/ml x 100 kg) /100 = .4 vials x 38 mg antibody/vial = 15.2 mg antibody. Neutralizing doses for DIGIBIND® and DIGIFAB™ may be readily determined by one of ordinary skill in the art using information and instructions provided in the product inserts for such compositions.

***"low dose"*** when used in connection with "digoxin antibody" or "digoxin antibodies" for treating a cytokine mediated condition, means a digoxin antibody composition having more digoxin binding capacity than a neutralizing dose, and less than 0.005 mg digoxin binding capacity per kg patient body weight. Non-limiting examples of low dose compositions are described in Tables I and II below.

***"high dose"*** when used in connection with "digoxin antibody" or "digoxin antibodies" for treating a cytokine mediated condition, means a digoxin antibody composition having 0.005 mg or greater digoxin binding capacity per kg patient body weight. Preferably, a "high dose" composition has digoxin binding capacity as provided in any of Tables III, IV or V.

***"mg"*** means milligram or milligrams.

***"µg" or "mcg"*** means microgram or micrograms.

***"pg"*** means picogram.

***"ng"*** means nanogram or nanogram.

***"kg"*** means kilogram or kilograms.

***"ml"*** means milliliter or milliliters.

***"Clinical Global Impression" or "CGI"*** means the clinical assessment of (1) the severity of a patient's condition at the time of evaluation ("CGI-S"), and/or (2) the improvement in a patient's condition since a prior evaluation or initial severity screen ("CGI-I"). The CGI is designed to document overall clinical impression of the patient's overall condition. See, Guy W., ECDEU Assessment Manual for Psychopharmacology - Revised, Rockville, Maryland, U.S. Dept. Health and Human Services [Publ No ADM 76-338] (1976, pgs. 218-222). Although the CGI-S and CGI-I scales described below are preferred, for purposes of the invention the original Guy scales or any modified form of a CGI-S and/or CGI-I scale may be used.

| **Severity Scale (CGI-S)** | | **Improvement (or Change) Scale** (**CGI**-**I**) | |
|---|---|---|---|
| 1. | Normal, not ill at all. | 1. | Very much improved. |
| 2. | Borderline ill. | 2. | Much improved. |
| 3. | Mildly ill. | 3. | Minimally improved. |
| 4. | Moderately ill. | 4. | No change. |
| 5. | Markedly ill. | 5. | Minimally worse. |
| 6. | Severely ill. | 6. | Much worse. |
| 7. | Among the most extremely ill patients. | 7. | Very much worse. |

### II. POTENTIAL ROLE OF THE SODIUM PUMP

Na⁺/K⁺ ATPase is a ubiquitous membrane-bound protein that functions to maintain a chemical and electrical gradient across the cell membrane by moving Na⁺ out and K⁺ into the cell, through the hydrolysis of ATP to ADP. The energy generated by Na⁺/ K⁺ ATPase drives other membrane transport, co-transport and exchange systems, allows the movement of cations, anions, amino acids and glucose across the cell membrane and maintains vital cellular functions including membrane electrical potential in excitable cells, regulation of osmotic balance and cell volume, and intracellular Na⁺ and Ca²⁺ concentrations. Na⁺/K⁺ ATPase activity will vary in response to changes in cellular environment in order to maintain low intracellular Na⁺ and the normal electrochemical gradient.

Any substance or physiological condition that alters the normal activity of Na⁺/K⁺ ATPase will have the effect of changing intracellular Na⁺ and Ca²⁺ and the electro-chemical gradient, leading to a variety of cellular dysfunctions. Na⁺/K⁺ ATPase is the only known receptor for cardiac glycosides, such as digitalis, ouabain and marinobufagenin. The presence of a binding site for exogenous cardiac glycosides suggests the existence of endogenous sodium pump inhibitors and their action in regulating the activity of Na⁺/K⁺ ATPase, intracellular sodium and calcium ion homeostasis, and thereby endogenous sodium pump inhibitors may regulate a variety of cellular functions. Abnormal sodium pump activity has been postulated to be involved in the pathophysiology of a variety of diseases, including cardiovascular, neurological, renal, and metabolic disorders.

It has been shown that ouabain stimulates proinflammatory cytokine gene expression (e.g., TNF and GM-CSF) in murine macrophages and human embryonal fibroblasts. See, Ohmori Y., et al., J. Cell Physiol. 148:96-105; Tamura M., et al., (1985) J. Biol. Chem. 260: 9672-9677. Ouabain inhibition of Na⁺/K⁺ ATPase also stimulates VCAM-1 gene expression and iNos expression. Bereta J., et al., FEBS Leters 377:21-25 (1995).

Furthermore, inhibition of Na⁺/K⁺ ATPase is known to cause detachment of cell monolayers and cell-to-cell junction detachment. Contreras RG, et al., J. Cell Sci. 112:4223-4231 (1999). Na⁺/K⁺ ATPase activity is required for E-cadherin mediated cell-to-cell adhesion in kidney epithelial cells, and for polarization, cell motility and suppression of invasion in those cells. Rajaskekaran SA., et al., Mol. Biol. Cell 12:270-295 (2001). It has been suggested that the level of Na⁺/K⁺ ATPase activity has a significant impact on cell attachment via the effect on intracellular Ca²⁺. Belusa R, et al., Am J. Physiol Cell Physiol 282:302-309 (2002).

Thus, it is theorized herein that one or more features of the inflammatory response, including endothelial activation and vascular permeability, may be mediated through Na⁺/K⁺ ATPase.

### III. SODIUM PUMP INHIBITORS

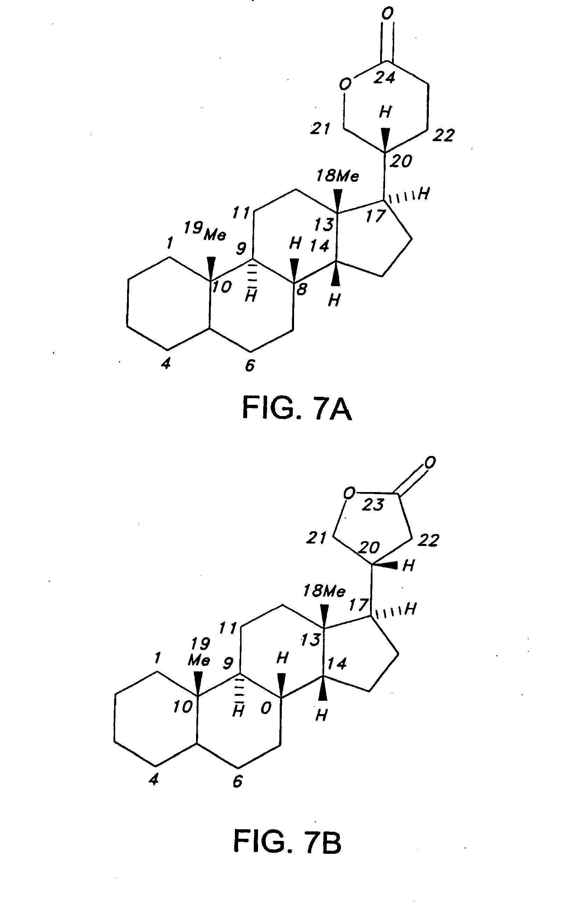

Cardiac glycosides (cardenolides and bufadienolides) are known to specifically inhibit Na⁺/K⁺ ATPase. They are composed of two structural features, the sugar (glycoside) and the non-sugar (aglycone) steroid moieties. The glycoside moiety may not significantly affect sodium pump binding affinity but may alter the biopharmaceutical or pharmacokinetic properties of the cardiac glycoside. The aglycone moieties of cardenolides and bufadienolides are known to be sodium pump inhibitors. Pullen MA, et al., J. Pharm. and Exp. Therapeutics 310(10): 319-325 (2004). Aglycones of the general class of cardenolides and bufadienolides are shown, respectively, in **Figs. 7A** and **7B****.**

Endogenous sodium pump inhibitors are found in patients having mineralocorticoid hypertension (primary aldosteronism and ectopic corticotrophin syndrome), essential hypertension, brain injury, and hypertension in plasma-volume expanded states including normotensive pregnancy and pregnancy complicated by pregnancy-induced hypertension (preeclampsia/eclampsia). These endogenous factors inhibit activity of sodium/potassium ATPase *in vitro* and cross-react with antibodies to exogenous sodium pump inhibitors, e.g. digoxin and ouabain. The endogenous factors have been variously described as "endoxin," "endobain," "digoxin-like," "digitalis-like," dihydrodigoxin, "endogenous ouabain," "ouabain-like," dihydroouabain, "dihydroouabain-like," "proscillaridin A-like," "endogenous marinobufagenin," "marinobufagenin-like," "bufalin-like" and 19-norbufalin. It is known that, *in vitro,* sodium pump inhibition by endogenous factors may be reversed or prevented by addition of antibodies to cardenolides and bufadienolides, particularly digoxin immune Fab. Pullen MA, et al., J. of Pharm. and Exp. Therapeutics 310(10): 319-325 (2004).

Digitalis or its constituents, digoxin and digitoxin, are the primary cardiotonic steroids that are used to treat cardiac arrhythmias, cardiac insufficiency and congestive heart failure. Digoxin and digitoxin have a narrow therapeutic ranges (1.0-1.9 nmol/L or approximately 0.8-1.5 ng/ml serum digoxin concentration) and overdose to these drugs is not uncommon. Digoxin overdose and life-threatening digoxin toxicity are treated through the administration of polyclonal digoxin immune Fab (ovine). It is believed that antibodies counteract the effects of digoxin or digitalis because the binding domain of the antibody binds to the cardiac glycoside thereby preventing it from inhibiting or regulating the expression or function of Na⁺/K⁺ ATPase. If the antibody is specific to the cardiac glycoside, the antibody will bind the cardiac glycoside with high affinity, favoring movement of the cardiac glycoside out of tissue and allowing the resulting antigen/antibody complex to be eliminated from the body.

### IV. THERAPEUTIC ANTIBODIES IN GENERAL.

As further described herein, it has been unexpectedly discovered that digoxin antibodies are effective for use in the treatment of localized or systemic inflammatory responses associated with or mediated or modulated by TNF. Digoxin antibodies of the present invention may be prepared as described below.

All naturally occurring whole antibodies have a common core structure of two identical light chains, each being about 24 kilodaltons, and two identical heavy chains each being about 55-70 kilodaltons. One light chain is attached to each heavy chain, and the two heavy chains are attached to each other. Both the light and heavy chains contain a series of repeating homologous units, each of about 110 amino acid residues in length which fold independently in a common motif called an immunoglobulin (Ig) domain. All Ig domains contain the complementarity determining regions ("CDRs"), which are specific for and bind to the antigen or epitope. There are between 10⁸ and 10¹⁰ structurally different antibody molecules in every individual. Antibody sequence diversity is predominantly found in three short amino acid sequences within the amino terminal variable domains of the heavy and light chains, called the hypervariable regions, to distinguish them from the more conserved "framework regions" that flank each CDR within the variable regions of the light and heavy chains.

Despite their overall similarity, antibody molecules can be divided into distinct classes and subclasses based on physiochemical characteristics such as size, charge and solubility, and on their behavior in binding to antigens. In humans, the classes of antibody molecules include IgA, IgD, IgE, IgG and IgM. Members of each class are said to be of the same isotype. IgA and IgG isotypes are further subdivided into subtypes called IgA₁, IgA₂ and IgG₁, IgG₂, IgG₃ and IgG₄. The heavy chains of all antibody molecules in an isotype share extensive regions of amino acid sequence homology, but differ from antibodies belonging to other isotypes. Heavy chains are designated to the overall isotype of the antibody molecule, e.g., IgA contains "alpha", IgD contains "delta", IgE contains "epsilon", IgG contains "gamma", and IgM contains "mu". IgG, IgE and IgD circulate as monomers. IgA circulates as a monomer, and molecules secreted through the epithelia into the mucosal lining of body cavities are homodimers. IgM molecules form pentamers.

Animals may be inoculated with an antigen in order to produce antibodies specific for the antigen. Frequently an antigen is bound or conjugated to another molecule to enhance the immune response. As used herein, a conjugate is any peptide, polypeptide, protein or non-proteinaceous substance bound to an antigen that is used to elicit an immune response in an animal. Antibodies produced in an animal in response to antigen inoculation comprise a variety of non-identical molecules (polyclonal antibodies) made from a variety of individual antibody producing B lymphocytes. A polyclonal antibody is a mixed population of antibody species, each of which may recognize a different epitope on the same antigen. Given the correct conditions for polyclonal antibody production in an animal, most of the antibodies in the animal's serum will recognize the collective epitopes on the antigenic compound to which the animal has been immunized. This specificity is further enhanced by affinity purification to select only those antibodies that recognize the antigen or epitope of interest.

A monoclonal antibody is a single species of antibody wherein every antibody molecule recognizes the same epitope because all antibody producing cells are derived from a single B-lymphocyte cell line. Hybridoma technology involves the fusion of a single B lymphocyte with an immortal myeloma cell (usually mouse myeloma). This technology provides a method to propagate a single antibody-producing cell for an indefinite number of generations, such that unlimited quantities of structurally identical antibodies having the same antigen or epitope specificity (monoclonal antibodies) may be produced. However, in therapeutic applications a goal of hybridoma technology is to reduce the immune reaction in humans that may result from administration of monoclonal antibodies generated by the non-human (e.g. mouse) hybridoma cell line.

Methods have been developed to replace light and heavy chain constant domains of the monoclonal antibody with analogous domains of human origin, leaving the variable regions of the foreign antibody intact. Alternatively, "fully human" monoclonal antibodies are produced in mice transgenic for human immunoglobulin genes. Methods have also been developed to convert variable domains of monoclonal antibodies to more human form by recombinantly constructing antibody variable domains having both rodent and human amino acid sequences. In "humanized" monoclonal antibodies, only the hypervariable CDR is derived from mouse monoclonal antibodies, and the framework regions are derived from human amino acid sequences. It is thought that replacing amino acid sequences in the antibody that are characteristic of rodents with amino acid sequences found in the corresponding position of human antibodies will reduce the likelihood of adverse immune reaction during therapeutic use. A hybridoma or other cell producing an antibody may also be subject to genetic mutation or other changes, which may or may not alter the binding specificity of antibodies produced by the hybridoma.

It is possible to create engineered antibodies, using monoclonal and other antibodies and recombinant DNA technology to produce other antibodies or chimeric molecules which retain the antigen or epitope specificity of the original antibody, i.e., the molecule has a binding domain. Such techniques may involve introducing DNA encoding the immunoglobulin variable region or the CDRs of an antibody to the genetic material for the framework regions, constant regions, or constant regions plus framework regions, of a different antibody. See, for instance, US Patent Nos. 5,091,513, and 6,881,557.

These binding fragments have binding specificity (CDR) for the antigen or epitope, but lack amino acid sequences in the conserved framework region, so they are less likely to elicit an immune response in a patient. An antigen binding fragment of the present invention preferably has a binding domain provided by one or more digoxin antibody variable domains and, most preferably, a binding domain of a digoxin antibody comprises an digoxin antibody light chain variable region (VL) and an digoxin antibody heavy chain variable region (VH).

Examples of binding fragments suitable for the present invention include, without limitation: (i) the Fab fragment, consisting of VL, VH, CL and CH1 domains; (ii) the "Fd" fragment consisting of the VH and CH1 domains; (iii) the "Fv" fragment consisting of the VL and VH domains of a single antibody; (iv) the "dAb" fragment, which consists of a VH domain; (v) isolated CDR regions; (vi) F(ab')2 fragments, a bivalent fragment comprising two linked Fab fragments; (vii) single chain Fv molecules ("scFv"), wherein a VH domain and a VL domain are linked by a peptide linker which allows the two domains to associate to form a binding domain; (viii) bi-specific single chain Fv dimers (see US Patent No. 5,091,513) and (ix) diabodies, multivalent or multispecific fragments constructed by gene fusion (US Patent App. Pub. 20050214860). Fv, scFv or diabody molecules may be stabilized by the incorporation of disulphide bridges linking the VH and VL domains. Minibodies comprising a scFv joined to a CH3 domain may also be made (Hu S., et al, Cancer Res., 56:3055-3061 (1996)).

By known means as described herein, polyclonal or monoclonal antibodies, binding fragments and binding domains and CDRs (including engineered forms of any of the foregoing), may be created that are specific to EFs, cardenolides and bufadienolides and their aglycone moieties, one or more of their respective epitopes, or conjugates of any of the foregoing, whether such antigens or epitopes are isolated from natural sources or are synthetic derivatives or variants of the natural compounds.

Antibodies may be produced from any animal source, including birds and mammals. Preferably, the antibodies are ovine, murine (e.g., mouse and rat), rabbit, goat, guinea pig, camel, horse, or chicken. In addition, newer technology permits the development of and screening for human antibodies from human combinatorial antibody libraries. For example, bacteriophage antibody expression technology allows specific antibodies to be produced in the absence of animal immunization, as described in US Patent No. 6,946,546, which is incorporated herein by this reference. These techniques are further described in: Marks, Bio/Technology 10:779-783 (1992); Stemmer, Nature 370:389-391 (1994); Gram et al., Proc. Natl. Acad. Sci., USA, 89:3576-3580 (1992); Barbas et al., Proc. Natl. Acad. Sci., USA, 91:3809-3813 (1994); and Schier et al., J. Mol. Biol. 263:551-567 (1996).

Methods for producing polyclonal antibodies in various animal species, as well as for producing monoclonal antibodies of various types, including humanized, chimeric, and fully human, are well known in the art and highly predictable. In addition, antibodies to various cardiac glycosides, including digoxin, ouabain, bufalin and marinobufagenin, are commercially available, and methods for producing these antibodies are also well known and predictable. For example, the following U.S. patents and patent applications provide enabling descriptions of such methods: U.S. Patent Application Nos. 2004/0126828 and 2002/0172677; and U.S. Patent Nos. 3,817,837; 3,850,752; 3,939,350; 3,996,345; 4,196,265; 4,275,149; 4,277,437; 4,366,241; 4,469,797; 4,472,509; 4,606,855; 4,703,003; 4,742,159; 4,767,720; 4,816,567; 4,867,973; 4,938,948; 4,946,778; 5,021,236; 5,164,296; 5,196,066; 5,223,409; 5,403,484; 5,420,253; 5,565,332; 5,571,698; 5,627,052; 5,656,434; 5,770,376; 5,789,208; 5,821,337; 5,844,091; 5,858,657; 5,861,155; 5,871,907; 5,969,108; 6,054,297; 6,165,464; 6,365,157; 6,406,867; 6,709,659; 6,709,873; 6,753,407; 6,814,965; 6,849,259; 6,861,572; 6,875,434; and 6,891,024.

It is fully expected that antibodies to a specific EF, cardenolide or bufadienolide will have the ability to neutralize or counteract the effects of the cardiac glycoside regardless of the animal species, monoclonal cell line or other source of the antibody. For example, although the antibody used in the Biological Examples of the present application was produced in sheep, it is expected that other animals immunized with the same or a similar cardenolide or bufadienolide would yield polyclonal antibodies effective for the purposes of the present invention. Certain animal species may be less preferable for generating therapeutic antibodies because they may be more likely to cause allergic response due to activation of the complement system through the "Fc" portion of the antibody. However, whole antibodies may be enzymatically digested into "Fc" (complement binding) fragment, and into binding fragments having the binding domain or CDR. Removal of the Fc portion reduces the likelihood that the antigen binding fragment will illicit an undesirable immunological response and, thus, antibodies without Fc may be preferential for prophylactic or therapeutic treatments. As described above, antibodies may also be constructed so as to be chimeric, partially or fully human, so as to reduce or eliminate the adverse immunological consequences resulting from administering to an animal an antibody that has been produced in, or has sequences from, another species.

It is known that EFs cross-react with or otherwise bind antibodies to exogenous cardenolides and bufadienolides, albeit in a less specific manner. Furthermore, it is known that antibodies to a specific cardenolide or bufadienolide will cross-react with other cardenolides and bufadienolides, although usually in a less specific manner. Thus, it is believed that any such cross-reactive antibody will have the ability to neutralize or counteract the effect of a non-specific cardiac glycoside or EF. For example, anti-marinobufagenin antibody is known to have cross-reactivity (expressed as a percentage) to various cardiotonic steroids as follows: marinobufagenin (100%); ouabain (0.1%); digoxin (1.0%); digitoxin (3.0%); bufalin (1.0%); proscillaridin (1.0%). Anti-ouabain antibody is known to have cross-reactivity to various cardiotonic steroids as follows: ouabain (100%); digitoxin (7.4%); proscillaridin (0.2%); marinobufagenin (0.5%); bufalin (0.03%). Anti-digoxin antibody is known to have cross-reactivity to various cardiotonic steroids as follows: digoxin (100%); ouabain (0.4%); oubagenin (0.1%); marinobufagenin (0.2%); bufalin (2.7%); cinobufotalin (4.3%); cinobufagin (0.02%). These examples of immunological cross-reactivity are not intended to limit in any manner the scope of the inventions disclosed herein.

If the non-specific antibody binding or cross-reactivity results in diminished antigen-antibody affinity or if the EF, cardenolide or bufadienolide has biopharmaceutical or pharmacokinetic characteristics that differs from the cardiac glycoside or EF that is specific for the antibody, then a greater amount of antibody may be required to neutralize or counteract the effects of the non-specific EF, cardenolide or bufadienolide. Nevertheless, it is fully expected and is known that, *in vitro,* antibodies to a specific cardenolide or bufadienolide will have the ability to counteract the sodium pump inhibition caused by a different cardenolide or bufadienolide or by one or more EFs. One of ordinary skill in the art may design appropriate antibody compositions and dosing regimens by know means, taking into account the level of sodium pump inhibition, the cross-reactivity, binding affinity or avidity, biopharmaceutical and/or pharmacokinetic properties of the specific as compared to the non-specific EF, cardenolide or bufadienolide.

Determination of an effective antibody composition to neutralize intoxication by an exogenous cardenolide or bufadienolide usually requires, *inter alia,* a determination of the body load of cardenolide or bufadienolide that must be bound or neutralized by the antibody. Factors that influence therapeutically effective antibody compositions include, *inter alia,* known or suspected total body load of antigen, whether it has reached steady-state equilibrium, bioavailability (free and protein bound), biopharmaceutical and pharmacokinetic properties of the antigen (e.g. antigen affinity for its receptor and bioactivity), patient weight or volume, history liver and renal function.

Generally, when an antigen reaches equilibrium within the body, antigen concentration (bound and unbound) in tissues and in extracellular fluids is reflected by the plasma or serum concentration. Total body load of the exogenous antigen generally equals the steady-state serum concentration of the antigen multiplied by the apparent volume of distribution (the fluid volume required to contain the antigen in the body at the same concentration as in plasma). Thus, the antibody composition generally required to neutralize the total body load may be determined from the binding capacity of the unit and the total body load of the antigen.

### V. DIGOXIN ANTIBODY COMPOSITIONS.

Proinflammatory cytokines (e.g., TNF) are not known to bind the sodium pump and are not known to have epitopes homologous to the sodium pump or any cardenolide or bufadienolide. Therefore, it would not be expected that digoxin antibodies would be capable of modulating or attenuating the action of any proinflammatory cytokine.

Surprisingly, it has been discovered that digoxin antibodies are effective in modulating or attenuating cell surface expression of various cell adhesion molecules that are necessary for inflammation, particularly for leukocyte-endothelial cell adhesion and transmigration of leukocytes into tissue. It is expected that, *in vivo,* modulating or attenuating the effects of TNF on cell adhesion molecule expression would have the corresponding effect of reducing leukocyte-endothelial cell interaction (capture, rolling and/or adhesion), thereby reducing leukocyte transmigration into tissue and reducing inflammation. Therefore, it is believed that digoxin antibodies may be useful and therapeutically effective for treating one or more cytokine mediated conditions.

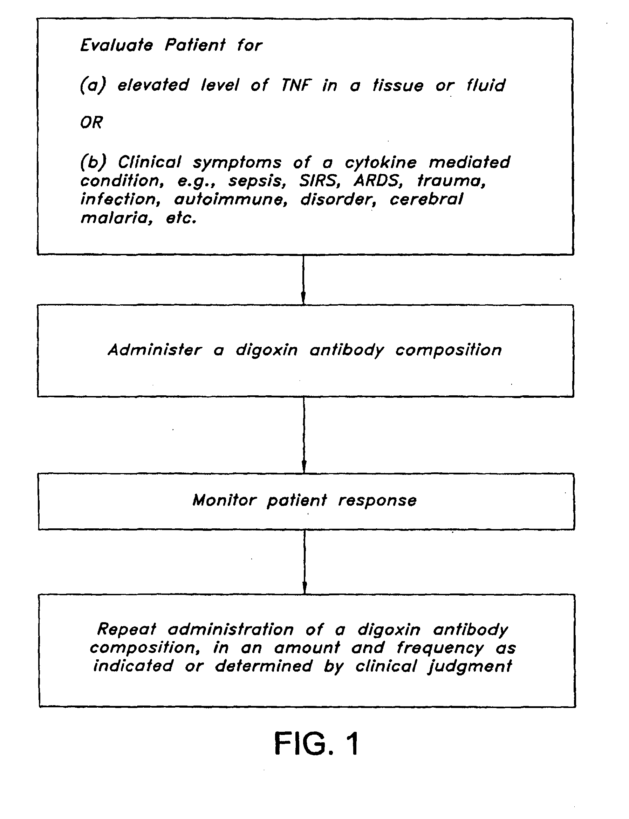

It is proposed that digoxin antibodies may be given to a patient in a therapeutically effective amount to attenuate expression of cell adhesion molecule that have been induced by TNF, according to the flow diagram of **Fig. 1****.** Similarly, if prior to onset of clinical or physiological symptoms of infection or pathology, a patient is determined to have a propensity for or to be at risk for developing a cytokine mediated condition or at risk for developing elevated levels of a proinflammatory cytokine, a therapeutically effective amount of digoxin antibody may be administered for prophylaxis to modulate the anticipated effects of TNF on cell adhesion molecule expression. While non-limiting examples have been provided herein, it should be emphasized that the present invention is not limited to any particular composition, dosage or range of dosages of digoxin antibodies, or digoxin antibodies of any particular type (e.g., polyclonal, monoclonal, chimeric, engineered) or derived from any particular source or sources (e.g. human, ovine, murine, etc.).

A therapeutically effective digoxin antibody composition modulates or attenuates TNF induction of cell surface expression of one or more cell adhesion molecules, including endothelial cell adhesion molecules that act as receptors for leukocyte adhesion ligands, and endothelial cell adhesion molecules that act as ligands to leukocyte adhesion molecule receptors. Preferably, a therapeutically effective digoxin antibody composition modulates or attenuates TNF-induced cell surface expression of ICAM-1, VCAM-1, P-selectin or E-selectin in one or more cell types, tissues and/or organs, preferably in endothelial cells and most preferably in vascular endothelial cells.

A therapeutically effective digoxin antibody composition may also increase Na⁺/K⁺ ATPase activity and/or Na⁺/K⁺ facilitated ion or nutrient transport in one or more cell types or tissues, and most preferably in the cells, tissues or organs adversely affected by the cytokine mediated condition, such as vascular endothelial cells and leukocytes. A therapeutically effective amount may also be an amount to increase Na⁺/K⁺ ATPase gene expression (e.g. increases in messenger ribonucleic acid (mRNA) corresponding to a sodium pump gene, which mRNAs are transcribed into polypeptides or proteins that form the sodium pump), or increase translocation to the cell membrane, or to decrease Na⁺/K⁺ ATPase degradation or recycling in particular cells, tissues or organs affected by the cytokine mediated condition.

A therapeutically effective digoxin antibody composition, when administered to a patient exhibiting symptoms of a cytokine mediated condition, will preferably provide a clinically beneficial effect, namely the alleviation, amelioration, reduction or inhibition of or improvement in one or more symptoms of the particular cytokine mediated condition, or improvement in the patient's general condition. A clinically beneficial effect may result in a beneficial change from baseline (i.e., before administration of digoxin antibody) for a specific medical parameter, and most preferably results in a reduction in CGI-S or a positive change in CGI-I for the patient. Alternatively, a therapeutically effective amount is an amount sufficient to stabilize a symptom of the cytokine mediated condition such that the symptom does not materially worsen.

Parameters that may be evaluated for or that may indicate therapeutic efficacy of a digoxin antibody composition (or other therapeutic agent) may include, without limitation: GCI-S or GCI-I; heart rate, respiration (e.g., rate or PaCO₂), systolic or diastolic blood pressure, or mean arterial pressure; tissue or organ perfusion; urinary output, urinary protein levels (proteinuria) or creatinine clearance; serum creatinine, lactate dehydrogenase (LDH), liver enzymes (e.g., alanine aminotransferase or aspartate aminotransferase), bilirubin or blood urea nitrogen (BUN) levels; leukocyte count; platelet count; red blood cell count or hemolysis; peripheral edema, pulmonary edema, cerebral edema or cerebral hemorrhage; pain, neurological function and/or neurological responsiveness, consciousness, mental status or neurological disturbances, aberrations or deficits; or any other medical parameter that may be evaluated in connection with the particular cytokine mediated condition.

The pharmaceutical compositions for use of the invention may be for use by administration to any animal which may experience the beneficial effects of the compositions of the present invention. Foremost among such animals are mammals, e.g., humans, although the invention is not intended to be so limited. Other mammals include: zoological animals, such as chimpanzees, monkeys, baboons, apes, tigers, lions, bears and the like; agricultural livestock, such as cows, sheep, pigs, goats and the like; and domestic animals, such as horses; dogs, cats and the like.

For therapeutic use, the digoxin antibody compositions may be for use by administration in any conventional dosage form in any conventional manner, all of which are well known in the art. The compositions of the invention may be for use by administration to a patient by any, or a combination of several, means including, without limitation, oral, topical, inhalation, intravenous, intrasynovial, transmucosal (e.g. nasal, vaginal, etc.), pulmonary, subcutaneous or intradermal or transdermal (injection or infusion or patch), ocular, buccal, sublingual, intraperitoneal, intrathecal, intramuscular or long term depot preparation, or any other means that is known in the art for administration of antibodies, binding fragments, binding domains, CDRs, peptides, calcyins or substances constructed with any of the foregoing.

For any form or method of administration, particularly for transmucosal, subcutaneous, intradermal, transdermal, intramuscular or intravenous administration (including intravenous bolus injections or bolus infusions), the composition may contain pharmaceutically acceptable carriers or excipients, including, without limitation, glucose, dextrose, saline, Ringer's or lactated Ringer's solution, Sterile Water for Injection, water or other pharmaceutically acceptable carriers or excipients, Preferably, the pH is suitably adjusted, the compositions are judiciously buffered and rendered isotonic, and sterilized by an appropriate method (e.g. by heating, irradiation or microfiltration) that is not detrimental to any component of the composition and that does not render the composition, or any component thereof, ineffective or less effective.

For use by intravenous administration, a loading dose may be administered by intravenous bolus injection or bolus infusion and may then be followed by a sustained intravenous infusion of a composition. Alternatively, a loading dose may be administered and then followed by subsequent bolus doses or a combination of bolus and sustained infusions may be employed. Similarly, a continuous infusion may be varied from time to time to administer greater or lesser amounts of digoxin antibody over a period of time.

The compositions for use of the invention may be for use by administration alone or in combination with adjuvants that enhance stability of the composition, facilitate administration of compositions containing them in certain embodiments, provide increased dissolution or dispersion, increase activity, provide adjunct therapy, and the like, including other active ingredients or other therapeutic agents. Advantageously, such combination therapies utilize lower dosages of the other therapeutic agents, thus avoiding possible toxicity and adverse side effects incurred when the other therapeutic agents are used as monotherapies. The above described digoxin antibody compositions may be physically combined with the other therapeutic agents or other adjuvants into a single pharmaceutical composition.

The composition of the digoxin antibody for use by administration to be therapeutically effective may depend upon a number of factors, for example: the pharmacodynamic characteristics of the particular therapeutic agent and its mode and route of administration; the type of patient (e.g., human, horse, dog, etc); the patient's physical condition, health (including presence of any disease, symptom or syndrome), gender, age and weight; the anticipated duration of treatment; the existence of predisposing factors for any disease, symptom or syndrome; the nature and extent of symptoms and the severity of the condition being treated; the patient response to one or more previously administered doses of the digoxin antibody and/or other therapeutic agents, or other compounds or compositions being concurrently administered; and the frequency of treatment of digoxin antibody and such other therapeutic agents, or compounds or compositions, and the effect desired.

The therapeutic composition comprises any amount of digoxin antibody, preferably at least a neutralizing dose of digoxin antibody, more preferably a low dose ditgoxin antibody composition and most preferably a high dose digoxin antibody composition. In one embodiment, the therapeutically effective composition comprises digoxin antibody sufficient to provide between 0.001 mg and approximately 500 mg digoxin binding capacity per kg patient body weight. In another embodiment, the composition comprises between approximately 0.005 and approximately 500 mgs digoxin binding capacity per kg patient body weight. In other , embodiments, the composition provides between approximately 0.005 and 50 mgs, or between 0.01 and 1.0 mg digoxin binding capacity per kg patient body weight, or between approximately 0.01 and 0.5 mg digoxin binding capacity per kg patient body weight.

Therapeutically effective digoxin antibody compositions may be readily determined by one of ordinary skill in the art by monitoring the patient for signs of absence, stabilization, amelioration, reduction, inhibition, or improvement in one or more indications, symptoms or complications of the cytokine mediated condition (e.g., the parameters described above), and correspondingly maintaining, increasing or decreasing the amount of digoxin antibody and/or frequency or manner of administration, as determined by clinical judgment.

The antibodies of the present invention may be produced by any of the various means described above (e.g., polyclonal, monoclonal, chimeric, humanized and other engineered antibody forms). Digoxin antibodies specific for various exogenous cardenolides and bufadienolides are well known in the art and are commercially available, including, without limitation, antibodies to digoxin, ouabain and marinobufagenin.

Because digoxin and digitoxin are the primary cardiotonic steroids that are used as therapeutic agents, antibody products to treat digoxin or digitoxin overdose, are well known in the art and have been specifically approved for therapeutic use in connection with potentially life-threatening digoxin and digitoxin intoxication. In rare instances, antibodies against digoxin or digitoxin (or conjugates thereof) have also been used "off label" to treat life-threatening intoxication by other exogenous cardenolides or bufadienolides. Morbidity and Mortality Weekly Report 44(46):853-855, 861 (Nov. 24, 1995); Eddleston, M. and Warrell, D. A., Q J Med. 92:483-485 (1999); *see, also,* Brubacher JR, Ravikumar PR et al., Chest 110:1282-1288 (1996); Brubacher JR, Lachmanen D, et al., Toxicon 37:931-942 (1999).

Digoxin immune Fab (ovine) compositions effective for treating life-threatening digoxin/digitoxin intoxication are currently marketed by GlaxoSmithKline in the United States under the brand name DIGIBIND^{®} and by Protherics, Inc. under the brand name DIGIFAB™. Digoxin immune Fab products effective for treating life-threatening digoxin/digitoxin intoxication may be produced and marketed outside the United States under other brand names. Clinical studies comparing DIGIBIND^{®} and DIGIFAB™ indicate that these products have equivalent pharmacokinetics and the proportion of patients responding to DIGIFAB™ were similar to, and consistent with, the historical data available for DIGIBIND^{®}.

DIGIBIND^{®} is a sterile lyophilized powder of antigen binding fragments (Fab) derived from specific antidigoxin antibodies raised in sheep. Production of DIGIBIND^{®} involves conjugation of digoxin as a hapten to human albumin. Sheep are immunized with this material to produce antibodies specific for the antigenic determinants of the digoxin molecule. The antibody is then papain-digested, and digoxin-specific Fab fragments of the antibody are isolated and purified by affinity chromatography. These antibody fragments have a molecular weight of approximately 46,200 Da. For the current formulation of DIGIBIND^{®}, one vial will bind approximately 0.5 mg of digoxin (or digitoxin), and contains 38 mg of digoxin-specific Fab fragments plus 75 mg of sorbitol as a stabilizer and 28 mg of sodium chloride. The vial contains no preservatives. DIGIBIND^{®} is administered by intravenous injection after reconstitution with Sterile Water for Injection (4 ml per vial), by gentle mixing, to give a clear, colorless, approximately isosmotic solution. Reconstituted product should be used promptly or it may be stored under refrigeration at 2° to 8°C (36° to 46°F) for up to 4 hours. The reconstituted product may be diluted with sterile isotonic saline to a convenient volume.

DIGIFAB™ is a sterile, purified, lyophilized preparation of digoxin-immune ovine Fab (monovalent) immunoglobulin fragments. These fragments are obtained from the blood of healthy sheep immunized with a digoxin derivative, digoxin-dicarboxymethoxylamine (DDMA), a digoxin analogue which contains the functionally essential cyclopentaperhydrophenanthrene lactone ring moiety coupled to keyhole limpet hemocyanin. The sheep are pathogen free and are from prion-free herds in Australia. The final product is prepared by isolating the immunoglobulin fraction of the ovine serum, digesting it with papain and isolating the digoxin-specific Fab fragments by affinity chromatography. These antibody fragments have a molecular weight of approximately 46,000 Da. For the current formulation of DIGIFAB™, one vial will bind approximately 0.5 mg digoxin and contains 40 mg of digoxin immune Fab, approximately 75 mg of mannitol USP, and approximately 2 mg sodium acetate (buffering agent). The product contains no preservatives and is intended for intravenous administration after reconstitution with 4 mL of Sterile Water for Injection USP. The reconstituted product may be added to an appropriate volume of 0.9% sodium chloride for injection.

DIGIBIND^{®} and DIGIFAB™ are not indicated for milder cases of digitalis (digoxin or digitoxin) toxicity and are only indicated for life-threatening or potentially life-threatening digoxin or digitoxin intoxication. Although designed specifically to treat life-threatening digoxin overdose, DIGIBIND^{®} and DIGIFAB™ have also been used successfully to treat life-threatening digitoxin overdose. However, since human experience is limited and the consequences of repeated exposures are unknown, DIGIBIND^{®} and DIGIFAB™ are not indicated for milder cases of digitalis toxicity. Clinical indications for FDA-approved administration of DIGIBIND^{®} and DIGIFAB™ do not include any condition other than known or suspected life-threatening digoxin or digitoxin intoxication. For purposes of the invention, life-threatening intoxication (sometimes referred to herein simply as "intoxication" or "intoxicated") with exogenous cardenolides and bufadienolides means: (a) fatal doses of 10 mg or more in previously healthy adults or 4 mg in previously healthy children; (b) ingestion causing steady-state serum concentrations greater than 10 ng/mL; or (c) chronic ingestions causing steady-state serum concentrations greater than 6 ng/mL in adults or 4 ng/mL in children. DIGIBIND^{®} and DIGIFAB™ will interfere with digitalis immunoassay measurements. Thus, standard measurement of serum digoxin (or "digoxin") concentration can be clinically misleading until the antibody is eliminated from the body. For treatment of intoxication, the composition of DIGIBIND^{®} or DIGIFAB™ depends upon the amount of digoxin or digitoxin to be neutralized.

However, cytokine mediated conditions are not caused by intoxication from exogenous cardenolides or bufadienolides. Cytokine mediated conditions are associated elevated levels of TNF and, thus, compositions and the methods of the present invention, provide that therapeutically effective digoxin antibody compositions for treating cytokine mediated conditions may be determined based upon the cytokine concentration in the patient. Direct measurements of cytokines in a tissue or body fluid may be performed by assays for detecting proinflammatory cytokines, including TNF, are well known in the art and commercially available (e.g., immunoassays provided by Bioveris, Alpco Diagnostics, Neogen). Therapeutically effective digoxin antibodies composition are preferably determined based upon the level of TNF-α in a patient's serum, plasma or tissue.

It should be noted that circulating (plasma or serum) levels of TNF in normal healthy humans or in healthy laboratory animals are estimated to be no more than approximately 10 pg/ml, a value that may be at the lower limit of detection by the most sensitive assays for TNF. Michie et al., New Eng. J. Med. 318:1481-1486 (1988); Mathison et al., J. Clin. Invest. 81:1925 (1988) and Waage et al., Lancet, 1:355-357 (1987). Following exposure to lipopolysaccharide ("LPS") a bacterial component associated with sepsis, the levels of TNF have been shown to increase 10-20 fold to approximately 400 pg/ml. Serum levels of TNF have been correlated with fatal outcome in infection by gram-negative (LPS-containing) meningococcal bacteria. Waage et al., Lancet, 1:355-357 (1987). Furthermore, similar increases in TNF were noted in subhuman primates models of sepsis and these changes were directly correlated with lethality. Tracey et al., Nature, 330:662-664, (1987). Thus, it is expected that TNF concentration (preferably TNF-α concentration) in a patient's tissue or body fluid will serve as an appropriate basis to select an effective digoxin antibody composition for therapeutic purposes.

Compositions within the scope of this invention include all compositions wherein the digoxin antibody compositions of the present invention are provided in an amount which is effective to achieve its intended purpose as described herein. While individual patient needs vary, determination of optimal ranges of therapeutically effective amounts of a digoxin antibody composition is within the ordinary skill of the art.

Examples of therapeutically effective neutralizing, low dose and high dose digoxin antibody compositions are provided herein. Preferably, compositions comprise an amount of digoxin antibody sufficient to provide 0.005 mgs or greater digoxin binding capacity per kg patient weight (e.g. a high dose digoxin antibody composition). Therapeutically effective compositions for prophylaxis may also have between 0.0005 and 0.005 mg digoxin binding capacity per kg patient weight (e.g., a low dose digoxin antibody composition).

Examples of pharmaceutical compositions of low dose and high dose digoxin antibodies appropriate for typical human weights are described in Tables I, II, III, IV and V. For human or other mammal weights that are less than 40 kg, an appropriate composition may be determined by dividing the patient's weight by 40 and multiplying the result by the digoxin binding capacity appropriate for a 40 kg patient according to Tables I-V. For animals with a weight that is greater than 140 kg, an appropriate composition may be determined by dividing the animal's weight by 100 and multiplying the result by a digoxin binding capacity within the range of digoxin binding capacities provided in Table I -V for a patient weighing 100 Kg. For example, if a patient weighs 500 kg and has a serum TNF concentration of 10 pg/mL, then an appropriate digoxin antibody composition according to Table IV is: 500/100 x 4.0 mg digoxin binding capacity = 20 mg digoxin binding capacity (or 40 vials of either DIGIBIND^{®} or DIGIFAB™ in the formulations described in the notes to Table V).

Table I identifies low dose digoxin antibody compositions for treating cytokine mediated conditions within the range of circulating TNF that has been observed in humans:

**Table I. Sample Low Dose* Digoxin Antibody Compositions for Cytokine Mediated Conditions (mg digoxin binding capacity)**

| **Patient Weight (kg)** | **TNF Concentration (pg/ml)** | | | | | | |
|---|---|---|---|---|---|---|---|
| | **10** | **25** | **50** | **100** | **200** | **300** | **400** |
| **40** | 0.02 | 0.04 | 0.08 | 0.16 | 0.24 | 0.32 | 0.4 |
| **60** | 0.03 | 0.06 | 0.12 | 0.24 | 0.36 | 0.48 | 0.6 |
| **70** | 0.035 | 0.07 | 0.14 | 0.28 | 0.42 | 0.56 | 0.7 |
| **80** | 0.04 | 0.08 | 0.16 | 0.32 | 0.48 | 0.64 | 0.8 |
| **100** | 0.05 | 0.10 | 0.20 | 0.40 | 0.60 | 0.80 | 1.0 |
| **120** | 0.06 | 0.12 | 0.24 | 0.48 | 0.72 | 0.96 | 1.2 |
| **140** | 0.07 | 0.14 | 0.28 | 0.56 | 0.84 | 1.12 | 1.4 |

| | | | | | | | |
|---|---|---|---|---|---|---|---|
| ***compositions may be suitable for prophylactic administration, non-systemic, chronic or episodic cytokine mediated conditions.** | | | | | | | |

Table II identifies low dose DIGLBIND^{®} of DIGIFAB™ compositions for treating cytokine mediated conditions within the range of circulating TNF have been observed in humans:

**Table II. Preferred Low Dose* DIGIBIND^{®} or DIGIFAB™ for Cytokine Mediated Conditions (in mg total antibody)**

| **Patient Weight (kg)** | **TNF Concentration (pg/ml)** | | | | | | |
|---|---|---|---|---|---|---|---|
| | **10** | **25** | **50** | **100** | **200** | **300** | **400** |
| **40** | 1.56 mg | 3.12 mg | 6.25 mg | 12.5 mg | 18.75 mg | 25.0 mg | 31.25 mg |
| **60** | 2.34 mg | 4.69 mg | 9.37 mg | 18.75 mg | 28.12 mg | 37.5 mg | 46.88 mg |
| **70** | 2.73 mg | 5.47 mg | 10.94 mg | 21.88 mg | 32.81 mg | 43.75 mg | 54.69 mg |
| **80** | 3.12 mg | 6.25 mg | 12.5 mg | 25.0 mg | 37.5 mg | 50.0 mg | 62.5 mg |
| **100** | 3.91 mg | 7.81 mg | mg 16.0 mg | 31.25 mg | 46.88 mg | 62.5 mg | 78.12 mg |
| **120** | 4.69 mg | 9.37 mg | 18.75 mg | 37.5 mg | 56.25 mg | 75.0 mg | 93.75 mg |
| **140** | 5.47 mg | 10.94 mg | 21.88 mg | 43.75 mg | 65.63 mg | 87.5 mg | 109.4 mg |

| | | | | | | | |
|---|---|---|---|---|---|---|---|
| *Compositions average 39 mg antibody/vial, 0.0128 mg digoxin binding capacity per mg total antibody. | | | | | | | |

Tables III identifies intermediate doses between Tables II and IV.

**Table III. High Dose Digoxin Antibody Compositions for Cytokine Mediated Conditions (mg digoxin binding capacity)**

| **Patient Weight (kg)** | **TNF Concentration (pg/mL)** | | | | | | |
|---|---|---|---|---|---|---|---|
| | **10*** | **25** | **50** | **100** | **200** | **300** | **400** |
| **40** | 0.25 | 0.40 | 0.50 | 0.60 | 0.70 | 0.80 | 1.0 |
| **60** | 0.40 | 0.45 | 0.50 | 0.55 | 0.75 | 1.0 | 1.2 |
| **70** | 0.45 | 0.50 | 0.55 | 0.65 | 0.85 | 1.2 | 1.4 |
| **80** | 0.55 | 0.55 | 0.60 | 0.75 | 1.0 | 1.3 | 1.6 |
| **100** | 0.65 | 0.75 | 0.80 | 0.80 | 1.2 | 1.6 | 2.0 |
| **120** | 0.80 | 0.85 | .90. | 1.0 | 1.5 | 2.0 | 2.4 |
| **140** | 0.90 | 1.2 | 1.3 | 1.5 | 1.8 | 2.4 | 2.8 |

| | | | | | | | |
|---|---|---|---|---|---|---|---|
| *Compositions are approximately 0.0065 mg digoxin binding capacity per kg. | | | | | | | |

**Table IV. Preferred High Dose Digoxin Antibody Compositions for Cytokine Mediated Conditions (mg digoxin binding capacity)**

| **Patient Weight (kg)** | **TNF Concentration (pg/mL)** | | | | | | |
|---|---|---|---|---|---|---|---|
| | **10*** | **25** | **50** | **100** | **200** | **300** | **400** |
| **40** | 0.5 | 0.5 | 1.0 | 1.5 | 2.5 | 3.5 | 4.0 |
| **60** | 0.5 | 0.5 | 1.5 | 2.5 | 3.5 | 5.0 | 6.0 |
| **70** | 0.5 | 1.0 | 1.5 | 3.0 | 4.5 | 5.5 | 7.0 |
| **80** | 0.75 | 1.0 | 1.5 | 3.5 | 5.0 | 6.5 | 8.0 |
| **100** | 0.75 | 1.0 | 2.0 | 4.0 | 6.0 | 8.0 | 10.0 |
| **120** | 1.0 | 1.0 | 2.5 | 5.0 | 7.0 | 9.5 | 12.0 |
| **140** | 1.0 | 1.5 | 3.0 | 5.5 | 8.5 | 11.0 | 14.0 |

Table V identifies high dose compositions of DIGIBIND® and DIGIFAB™ that correspond to the compositions of Table IV.

**Table V. Preferred High Dose DIGIBIND^{®}/ DIGIFAB ™ For Cytokine Mediated Conditions (in # of ("V") vials)**

| **Patient Weight (kg)** | **TNF Concentration (pg/ml)** | | | | | | |
|---|---|---|---|---|---|---|---|
| | **10*** | **25** | **50** | **100** | **200** | **300** | **400** |
| **40** | 1 V | 1 V | 2 V | 3 V | 5 V | 7 V | 8 V |
| **60** | 1 V | 1 V | 3 V | 5 V | 7 V | 10 V | 12 V |
| **70** | 1 V | 2 V | 3 V | 6 V | 9 V | 11 V | 14 V |
| **80** | 1.5 V | 2 V | 3 V | 7 V | 10 V | 13 V | 16 V |
| **100** | 1.5 V | 2 V | 4 V | 8 V | 12 V | 16 V | 20 V |
| **120** | 2 V | 3 V | 5 V | 10 V | 15 V | 20 V | 24 V |
| **140** | 2 V | 3 V | 6 V | 12 V | 17 V | 23 V | 28 V |

| | | | | | | | |
|---|---|---|---|---|---|---|---|
| *Compositions average 39 mg antibody/vial, providing an average of 0.0128 mg digoxin binding capacity / mg total antibody. | | | | | | | |

The compositions of Tables I, II, IIII, IV and V are preferred compositions only, and compositions that may be therapeutically effective for any particular patient weight or circulating or localized cytokine (e.g., TNF) concentration may have digoxin binding capacity that is greater than or less than the stated preferred amounts. For TNF concentrations falling between the specific concentrations identified in Tables I, II, III, IV or V, in most cases the composition should be given in an amount that is intermediate between the amounts for the next lower and next higher concentration for the patient's weight. Similarly, for patient weights falling between those listed in Tables I, II, III, IV and V, in most cases the dose should be given for the next highest weight for the particular TNF concentration. However, if an intermediate amount may be obtained, the intermediate amount is preferable. For example, if serum TNF concentration (whether measured or suspected to be present based upon severity of one or more symptoms and known concentrations in other patients with similar symptoms or conditions) is 100 pg/ml for a patient weight of 115 kg, a therapeutic composition comprising 9 vials of DIGIBIND^{®} or DIGIFABT"' (Table V) would be administered as a single application dose.

For digoxin antibody formulations (i.e., dosing units), including, without limitation, DIGIBIND^{®} and DIGIFAB™, that have greater or lesser digoxin binding capacity than the DIGIBIND^{®} and DIGIFAB™ formulations of Table II or V (current formulation 0.5 mg digoxin binding capacity per vial), antibody compositions should be adjusted to provide digoxin binding capacity that is equivalent to the digoxin binding capacity of the compositions (# vials) described in Table II or V.

Preferably, the TNF concentration should be obtained from a tissue or body fluid (preferably plasma or serum) before initial administration of digoxin antibody. Although it is preferable to measure TNF concentration before administration of digoxin antibody, it is not necessary to do so in order to determine a therapeutically effective composition. If TNF concentration cannot be readily quantified, the patient initially may be given a composition commensurate with the severity of the symptoms or the particular cytokine mediate condition. Generally, mild symptoms initially may be treated with a therapeutic neutralizing dose or low dose composition, for example according to Table I. or Table II, or a dose corresponding to a lower cytokine concentration (e.g. TNF concentration of 10 pg/ml or 25 pg/ml) from Tables III, IV or V. Generally, compositions for mild symptoms and prophylactic treatment are initially selected from a neutralizing dose or the lower range of low dose compositions of Tables I or II. In all circumstances, patients should be monitored throughout the course of treatment for improvement, attenuation, amelioration, stabilization or worsening of symptoms or complications. If the patient response to a digoxin antibody composition is adequate in the clinical judgment of the treating physician, then the composition may be repeated as needed to maintain the desired response. If an adequate response is not achieved with a composition or a greater response is desired, then a composition having a greater amount of the active ingredient (digoxin binding capacity) should be given in one or more subsequent administrations.

More severe symptoms initially may be treated in the mid-range of the compositions for the patient's weight according to Tables I - V, Again, the patient should be routinely monitored for amelioration, attenuation, improvement, stabilization or worsening of symptoms or complications, so that the composition may be adjusted (active ingredient decreased or increased) as determined by clinical judgment. For illustration purposes only, and without limitation, a 70 kg patient with symptoms of severe sepsis may be initially administered 3 vials of DIGIBIND^{®} or DIGIFAB™ or digoxin antibody of another formulation having approximately 1.5 mg digoxin binding capacity. In an alternate example, if septic shock is present, the patient may be initially administered a composition of between 4 and 6 vials of DIGIBIND^{®} or DIGIFAB™ or a digoxin antibody composition of another formulation or antibody species having between approximately 2.0 and 3.0 mg digoxin binding capacity.

For example, patients with severe sepsis are known to have serum concentrations of TNF in the range of 50 pg/mL to 400 pg/ml and a dose may be selected from the appropriate range of doses described in Tables I-V to treat severe sepsis in a patient. For purposes of the invention a TNF concentration may also be selected based upon concentrations generally known to be present in patients having the cytokine mediated condition that is being treated. Most preferably, the TNF concentration will be based upon the range generally known to be present in the particular cytokine mediated condition, will be selected based upon the state of patient, risk factors for cytokine mediated conditions, and/or symptom severity and/or complications of the patient being treated.

While certain of the examples provided herein are specific for current formulations of DIGIBIND^{®} or DIGIFAB™, it should be emphasized that the present invention is not limited to digoxin-immune Fab (ovine), DIGIBIND^{®} or DIGIFAB™, or to any particular brand or formulation of digoxin antibody, but encompasses all digoxin antibodies, including antigen binding fragments, and engineered antibodies or constructed molecules that contain a CDR or binding domain. Further the invention is not limited to any particular digoxin antibody composition (dose), dosing method or regimen or manner of administration.

The compositions of the invention comprise a single application dose. A single application dose includes the composition when given (a) entirely in a single administration or (b) when given in aliquots as multiple administrations over a period of time, or (c) when administered continuously over a period of time (e.g., continuous infusion, slow or time release transdermal administration, slow-release or time-release tablets or capsules or caplets).

Preferably, the patient is initially given a therapeutic composition having the lowest amount of active ingredient (digoxin binding capacity) appropriate for the patient according the dosing parameters described herein or as determined by clinical judgment and on having ordinary skill in the art. Thereafter, one or more subsequent compositions having greater or lesser amounts of the active ingredient may be administered to achieve or maintain the desired improvement in symptoms or complications of the condition being treated. However, in situations where the patient's symptoms or complications are potentially life-threatening, it may be preferable to initially administer a therapeutic composition having a greater amount of the active ingredient, preferably a high dose digoxin antibody composition.

The digoxin antibody of the composition is any antibody, binding fragment or any molecule having a binding domain that reacts immunologically with or binds a cardenolide, a bufadienolide or an EF, and preferably an antibody, binding fragment or binding domain that is specific for a cardenolide or a bufadienolide, including, without limitation, anti-ouabain antibody, anti-marinobufagenin antibody, anti-bufalin antibody and anti-digoxin antibody (e.g. DIGIBIND^{®} or DIGIFAB™).

Methods for quantifying relative cross-reactivity of an antibody for a different antigen or epitope are well known to those having ordinary skilled in the art. See, for example, Fedorova et al., Journal of Hypertension 23(4):835-842 (2005), and references therein. Generally, if an antibody is known to cross-react with digoxin, the cross-reactivity provides a basis to formulate a composition of such antibody to provide the digoxin binding capacity in a therapeutically effective amount as described above. For example, if a formulation of marinobufagenin antibody has 0.5 mg marinobufagenin binding capacity and a cross-reactivity to digoxin of 10%, then the same composition will have approximately 0.05 mg digoxin binding capacity.

The composition may be comprised of whole digoxin antibody, binding fragments, binding domains, CDRs or a combination thereof. The digoxin antibody may be polyclonal, monoclonal, chimeric, humanized or fully humanized, or other form of engineered antibody. The digoxin antibody of the invention may be generated from any known antibody source, including, without limitation, sheep, goat, horse, chicken, rabbit, mouse, mammalian cells lines, bacteria or yeast. The digoxin antibody source is preferably ovine (produced in sheep, or generated or constructed, in whole or in part, from polypeptides, proteins or nucleic acids of ovine origin). Preferably, the digoxin antibody is digoxin immune Fab and most preferably digoxin immune Fab (ovine).

While the above therapeutic compositions have been provided, deviations or modifications may be used. Furthermore, the compositions simply define a neutralizing dose as a dose for initial treatment, compositions having lesser amounts, intermediate amounts or greater amounts of digoxin binding capacity than the compositions provided herein, including those provided in Tables I, II, III, IV and V, are also expected to be effective and are also encompassed within the present invention.

Certain embodiments of the invention provide for use by administration of a digoxin antibody composition to a patient in need thereof for treating a cytokine mediated condition. Preferably, the composition is repeatedly administered from time to time and in such amount as is then needed. In certain embodiments, the composition is for use by administration on a fixed schedule, e.g., hourly intervals (e.g., every 2, 4, 6, 12 hours), daily, weekly, bi-weekly, monthly, or intermediate intervals thereof.

In one embodiment, compositions for use of the present invention are for use in treating severe hemorrhage and systemic or local inflammatory diseases and conditions, such as hemorrhagic shock, sepsis, septic shock, septicemia, Systemic Inflammatory Response Syndrome (SIRS), Compensatory Anti-Inflammatory Response Syndrome (CARS), multiple organ dysfunction syndrome (MODS), multiple organ failure (MOF), asthma, allergy, anaphylactic shock, immune complex disease, organ ischemia, reperfusion injury, organ necrosis, hay fever, endotoxic shock, cachexia, hyperpyrexia, eosinophilic granuloma, granulomatosis, and sarcoidosis.

In an alternate embodiment, compositions for use of the present invention may be for use in treating autoimmune and inflammatory conditions and disorders, such as anti-phospholipid syndrome, myasthenia gravis, thyroiditis, systemic lupus erythematosus, Goodpasture's syndrome, Behcet's syndrome, allograft rejection, graft-versus-host disease, hyperimmunoglobulinemia-D syndrome (HIDS), TNF-receptor associated periodic syndrome (TRAPS), pancreatic beta-cell destruction, insulin resistance, Type I and Type II diabetes, gestational diabetes mellitus, gestational insulin resistance, Berger's disease, and Reiter's syndrome.

In yet other embodiments, compositions for use of the present invention may be for use in treating diseases and disorders involving the blood, lymphatic and cardiovascular systems and associated tissues, such as tissue or organ ischemia, reperfusion injury, vasculitis, lymph edema, angiitis, endocarditis, arteritis, atherosclerosis, thrombosis and thrombophilia, thrombophlebitis, pericarditis, congestive heart failure, myocarditis, myocardial ischemia, ischemic stroke, periarteritis nodosa, restenosis, chemotherapy related anemia, and rheumatic fever.

In still other embodiments, compositions for use of the present invention may be for use in treating diseases and disorders involving the gastrointestinal tract and associated tissues, such as appendicitis, peptic, gastric and duodenal ulcers, peritonitis, pancreatitis, ulcerative colitis, pseudomembranous colitis, acute and ischemic colitis, diverticulitis, periodontal disease, epiglottitis, achalasia, cholangitis, cholecystitis, celiac disease, hepatitis, cirrhosis, inflammatory bowel disease, Crohn's disease, enteritis, and Whipple's disease.

In yet other embodiments, compositions for use of the present invention may be for use in treating diseases and disorders involving the urogenital system and associated tissues, such as spontaneous preterm labor, placental abruption, recurrent fetal loss, septic abortion, epididymitis, vaginitis, prostatitis, glomerulonephritis and urethritis.

In alternate embodiments, compositions for use of the present invention may be for use in treating diseases and disorders involving the respiratory system and associated tissues, such as bronchitis, emphysema, rhinitis, cystic fibrosis, pneumonia, pneumonitis, chronic obstructive pulmonary disease (COPD), adult (acute) respiratory distress syndrome (ARDS), pneumoultramicroscopicsilico-volcanoconiosis, alveolitis, bronchiolitis, pharyngitis, pleurisy, and sinusitis).

In still other embodiments, compositions for use of the present invention may be for use in treating infection and diseases arising from infection by various viruses, bacterial, fungi, protozoal and multicellular parasites, such as malaria (e.g. cerebral malaria), influenza viruses, respiratory syncytial viruses, Human Immunodeficiency Viruses (HIV), hepatitis viruses, herpes viruses, cytomegalovirus, meningitis and adenovirus, infections caused by gram positive and gram negative bacteria, tuberculosis and leprosy.

In other embodiments, compositions for use of the present invention may also be for use in treating dermatological diseases and conditions of the skin, such as psoriasis, allergic and acute dermatitis, actinic keratosis, dermatomyositis, chemical and other bums, sunburn, urticaria warts, and wheals.

In yet other embodiments, compositions for use of the present invention may be for use in treating neuropathic pain and diseases and conditions involving the central or peripheral nervous system and associated tissues, such as Alzheimer's disease, meningitis, encephalitis, demyelinating diseases, multiple sclerosis, myasthenia gravis, cerebral infarction, cerebral embolism, Guillame-Barre syndrome, neuritis, neuralgia, spinal cord injury, brain injury, paralysis, and uveitis.

In still other embodiments, compositions for use of the present invention may be for use in treating diseases and conditions of the bones, joints, muscles and connective tissues, such as the various arthritides and arthralgias, bone resorption diseases, muscle degeneration, anorexia, cachexia, osteoporosis, osteomyelitis, fasciitis, Paget's disease, gout, periodontal disease, rheumatoid arthritis, osteoarthritis, juvenile chronic arthritis (JCA), scleroderma, ankylosing spondylitis, and synovitis);

In other embodiments, compositions for use of the present invention also may be for use in treating diseases and disorders involving various cancers, tumors and proliferative disorders, such as Hodgkin's disease, multiple myeloma, acute and chronic myelogenous leukemia), metastasis, recurrence of cancers and tumors, and injury or trauma resulting from surgical, chemotherapy and radiation treatment of such cancers, tumors and proliferative disorders.

Because the innate and adaptive immune responses have redundant regulatory mechanisms, it is believed that attenuation or inhibition of one mechanism may not necessarily provide a sufficient therapeutic effect. Therefore, certain embodiments of the present invention provide for use by administering a therapeutically effective amount of digoxin antibody composition and at least one other therapeutic agent. The other therapeutic agent may be any therapeutic agent that has been used, is currently used, or is known or may become known to be useful for treating, ameliorating, or preventing a condition, infection, disorder, disease or pathology encompassed by the present invention. For a more detailed description of therapeutic agents, those skilled in the art are referred to instructive manuals including, but not limited to, *The Physician's Desk Reference* and to Goodman and Gilman's "Pharmaceutical Basis of Therapeutics" (ninth edition, McGraw Hill, 1996).

The digoxin antibody composition may be for use by administration at different periodicities, at different durations, at different concentrations, by different administration routes, etc. from another therapeutic agent or for a different cytokine mediated condition. For example, the digoxin antibody composition may be for use by administration prior to another therapeutic agent, e.g., without limitation, 0.5, 1, 2 3, 4, 5, 6, 8, 10, 12, 14, 16 or 18 hours, 1, 2, 3, 4, 5, or 6 days, 1, 2, 3, or 4 weeks prior to the administration of the other therapeutic agent. The digoxin antibody composition may also be for use by administration after another therapeutic agent, e.g., 0.5, 1, 2 3, 4, 5, 6, 8, 10, 12, 14, 16, or 18 hours, 1, 2, 3, 4, 5, or 6 days, 1, 2, 3, or 4 weeks after the administration of the other therapeutic agent.

Alternatively, the digoxin antibody composition and the other therapeutic agent are for use by administration concurrently but on different schedules, e.g., the digoxin antibody composition is administered periodically during a day, while the other therapeutic agent is administered daily. The digoxin antibody composition may also be for daily administration, while the other therapeutic agent is for use by less frequent than daily administration, such as once a week, once every two weeks, once every three weeks, or once every four weeks. The digoxin antibody composition may be for use by administration less frequently than daily (e.g. once a week), while the other therapeutic agent is for periodical administration throughout a day, daily, once a week, once every two weeks, once every three weeks, or once every four weeks. Any other use by periodic administration of the digoxin antibody and the other therapeutic agent is also suitable.

The combination of a compound for use of the invention and one or more other therapeutic agents can have additive potency or an additive therapeutic effect. The invention also encompasses synergistic combinations where the therapeutic efficacy is greater than additive. Preferably, such combinations also reduce or avoid unwanted or adverse effects. In certain embodiments, the combination therapies encompassed by the invention provide an improved overall therapy relative to administration of a digoxin antibody composition, or any other therapeutic agent alone. In certain embodiments, doses of existing or experimental other therapeutic agents may be reduced or administered less frequently when given in combination with the digoxin antibody composition of the present invention, which may increase patient compliance, thereby improving therapy and reducing unwanted or adverse effects.

For one or more embodiments of the invention, other therapeutic agents may also be for use by co-administration with or used in combination with one or more non-steroidal anti-inflammatory drugs (NSAIDS) such as piroxicam, naproxen, indomethacin, ibuprofen and the like; COX-2 selective inhibitors such as rofecoxib, which is available as Vioxx^{®} (from Merck & Company) and celecoxib, which is available as Celebrex^{®} (from Pfizer Inc.); COX-1 inhibitors such as Piroxicam, which is available as Feldene^{®} (from Pfizer Inc.); immunosuppressives such as described in U.S. Pat. 7,012,060, corticosteroids, cyclosporine, Tacrolimus, rapamycin, methotrexate and the like; biological response modifiers, such as TNF-antagonists or TNF-receptor antagonists, e.g. adalimumab, available as Humira^{®} (Abbott Laboratories), etanercept, which is available as Enbrel^{®} (from Wyeth-Ayerst), infliximab, which is available as Remicade^{®} (from Centocor, Inc.), Cytofab™ (polyclonal anti-TNF Fab (ovine) available from Protherics), IL-1 antagonists, anti-CD40, anti-CD28, IL-10, anti-adhesion molecules and the like, such as Tysabri^{®} (from Biogen Idec and Elan Pharmaceuticals), drotrecogin alpha (recombinant human activated Protein C), which is available as Xigris^{®} (Eli Lilly and Company), beta.2-receptor agonists (e.g., albuterol), leukotriene receptor antagonists (e.g., montelukast), and other antithrombotic or anti-inflammatory agents such as described in U.S. Patent Nos. 7,057,022 and 7,005,413, p38 kinase inhibitors, PDE4 inhibitors, TACE inhibitors, chemokine receptor antagonists, thalidomide, which is available as Thalomid^{®} (Celgene Corporation) and other small molecule inhibitors of pro-inflammatory cytokine production, agents used to treat autoimmune disease (e.g. cyclosporine, tacrolimus, mycophenolate mofetil); agents used to treat nervous system disorders (e.g., anticholinesterases, dopamine, levodopa, serotonin receptor agonists (e.g., sumatriptan, amantadine, donepezil, riluzole), agents used to treat ischemia/reperfusion injury (e.g., nitroglycerin, nifedipine), agents used to treat gastrointestinal disorders (e.g., neostigmine, metoclopramide, sulfasalazine) and agents used to treat ARDS, sepsis, SIRS, septic shock or hemorrhagic shock (e.g. described in U.S. Patent 6,193,969 and US Patent App. Pub. 2005/0187181).

Other therapeutic agents that the digoxin antibody compositions the invention may be for use by co-administration or for combined use with the compositions for use of the present invention include, without limitation, Anaprox^{®} (i.e., naproxen sodium), Arava^{®} (i.e., leflunomide), Arthrotec^{®} (i.e., combination of diclofenac and misoprostol), Azulfidine^{®} (i.e., sulfasalazine), aspirin (i.e., acetylsalicylic acid), Cataflam^{®} (i.e., diclofenac), Celestone^{®} Soluspan^{®} (i.e., betamethasone acetate and betamethasone sodium phosphate), Clinoril^{®} (i.e., sulindac), Cortone Acetate^{®} (i.e., cortisone acetate), Cuprimine^{®} (i.e., penicillamine), Daypro^{®} (i.e., oxaprozin), Decadron® (i.e., dexamethasone), Depen® (i.e., penicillamine), Depo-Medrol® (i.e., methylprednisolone acetate), Disalcid^{®} (i.e., salsalate), Dolobid^{®} (i.e., diflunisal), Naprosyn^{®} (i.e., naproxen), Gengraf^{®} (i.e., cyclosporine), Hydrocortone^{®} (i.e., hydrocortisone), Imuran^{®} (i.e., azathioprine), Indocin^{®} (i.e., indomethacin), Lodine^{®} (i.e., etodolac), Motrin^{®} (i.e., ibuprofin), Myochrysine^{®} (i.e., gold sodium thiomalate), Nalfon^{®} (i.e., fenoprofen calcium), Naprelan^{®} (i.e., naproxen sodium), Neoral^{®} (i.e., cyclosporine), Orudis^{®} (i.e., ketoprofen), Oruvail^{®} (i.e., ketoprofen), Pediapred^{®} (i.e., prednisolone), Plaquenil^{®} (i.e., hydroxychloroquine), Prelone^{®} (i.e., prednisolone), Relafen^{®} (i.e., nabumetone), Solu-Medrol^{®} (i.e., methylprednisolone sodium succinate), Tolectin^{®} (i.e., tolmetin sodium), Trilisate^{®} (i.e., choline magnesium trisalicylate) and Volataren^{®} (i.e., diclofenac). These include any formulation of the above named therapeutic agents. Dosing amounts and regimens for administering these agents are well known in the art.

It will be apparent to those of ordinary skill in the art that the use by administration of other therapeutic agents and/or other active agents in combination with the compositions for use of the present invention can be varied depending on the cytokine mediated condition being treated and the known effects of the agents on that condition. Also, in accordance with the knowledge of the skilled clinician, the therapeutic protocols (e.g. dosage amounts, modes and times of administration) can be varied in view of the observed effects of the administered agents on the patients, and in view of the observed responses of the cytokine mediated condition to the administered agents.

While the above compositions and combinations have been provided, deviations or modifications may be used, and the invention is deemed to include digoxin antibody compositions for use in treating TNF mediated conditions that have digoxin binding capacity that is less than, greater than, or is intermediate among the examples provided herein.

### VIII. BIOLOGICAL AND PROPHETIC EXAMPLES. The uses of the invention are further illustrated in the following non-limiting examples.

Human umbilical veins are readily available source of endothelial tissue and endothelial cells (referred to herein as "HUVECs"), and human umbilical veins and HUVECs are appropriate models for investigating endothelial cell activation and dysfunction in connection with the inventions disclosed herein. It is believed that the data and prophetic examples below demonstrate that the digoxin antibodies have a modulating or attenuating effect in more than one biological activities. As a consequence, the digoxin antibody compositions may be useful in preventing or treating more than one TNF-mediated condition. Because cell signaling pathways are similar for many cytokines, it is also expected that digoxin antibody compositions may be useful in treating inflammatory conditions induced by other proinflammatory cytokines (e.g., IL-1, IL-6, IL-8, IFN-γ, etc.) and other endogenous proinflammatory substances.

### Biological Example #1.

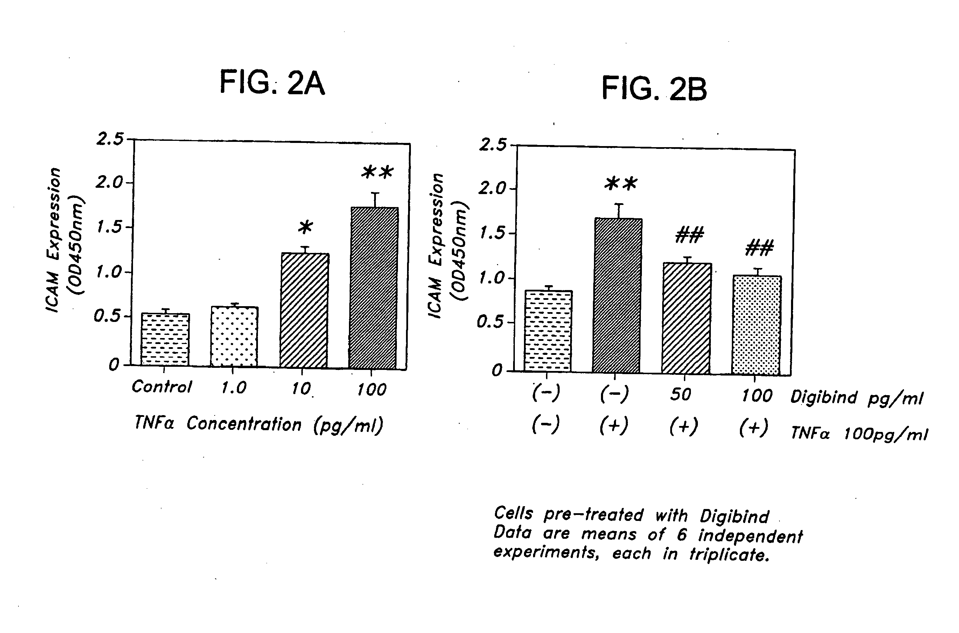

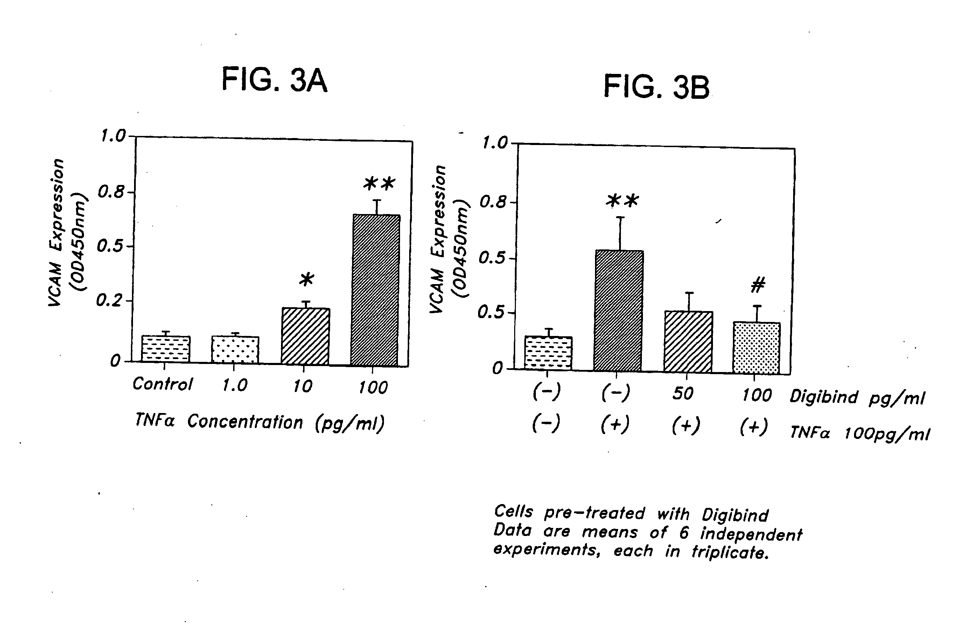

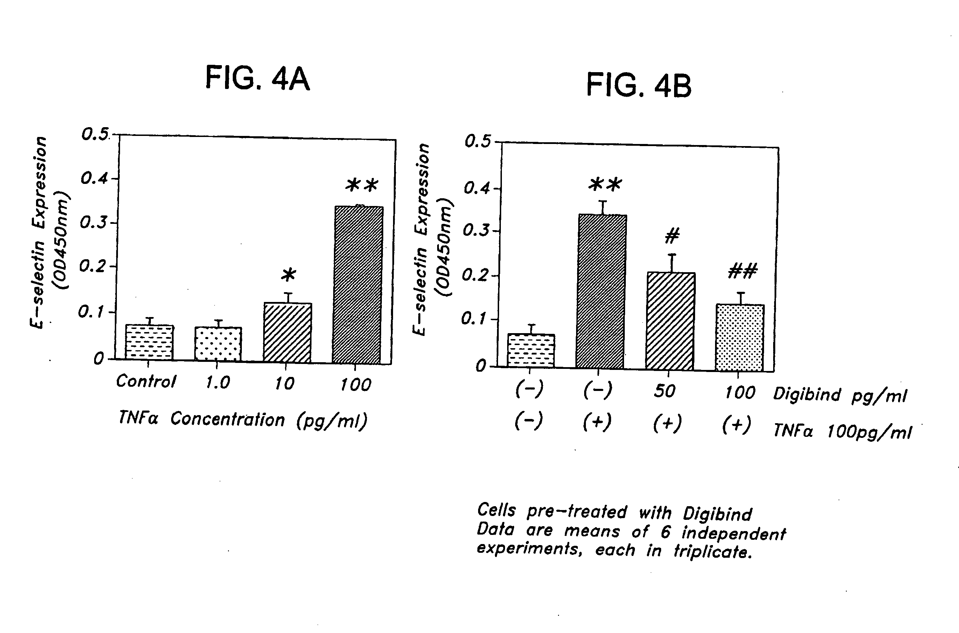

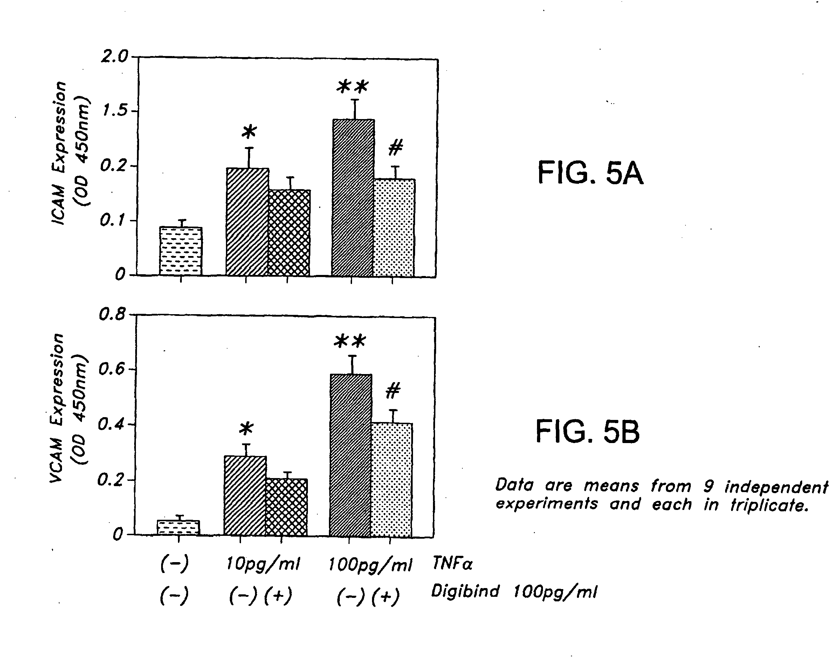

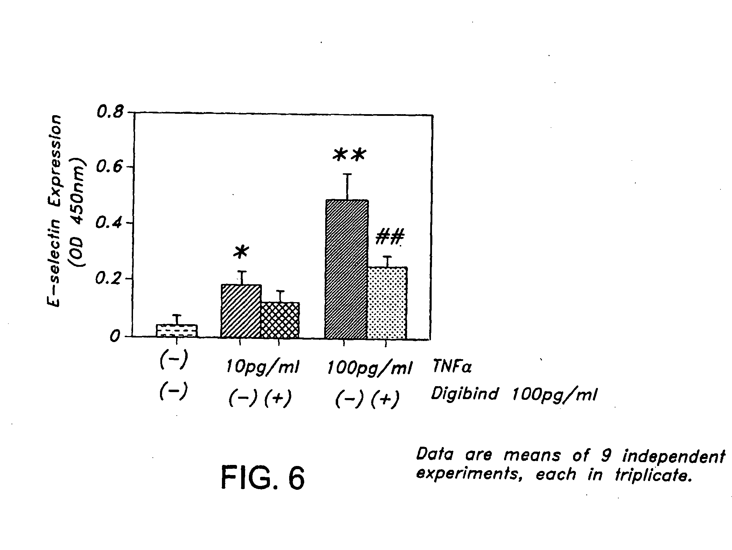

Endothelial cells (ECs) were isolated from umbilical cord vein from normal pregnant deliveries and cultured as described in Wang Y, Yang G and Lucas MJ, Expression of Thrombin Receptors in Endothelial Cells and Neutrophils from Normal and Preeclamptic Pregnancies, J. Clin. Endocrin. & Met. 87(8):3728-3724 (2002). Confluent ECs were grown in 48well/cluster cell culture plates and treated with TNF-α at concentrations of 1, 10, and 100pg/ml for 2 hours (TNF-α concentration is in a range of 50-100pg/ml in sepsis). EC activation was determined by immunoassay directly to detect EC surface expression of adhesion molecules ICAM, VCAM, and E-selectin. Data are expressed as mean ± S.D. and analyzed by ANOVA. Student-Newman-Keuls' test was used as post-hoc test. A"P" level of less than 0.05 was deemed to be statistically significant. **Figures 2A****,** **3A** and **4A** illustrate the increasing, dose-dependent TNF-α induction of cell surface expression of ICAM, VCAM and E-selectin, respectively. If ECs are pre-treated with digoxin antibody for one (1) hour prior to induction with TNF-α, then ICAM, VCAM and E-selectin expression is modulated, as shown in **Figures 2B****,** **3B** and **4B****,** suggesting that digoxin antibody exerts a protective effect on ECs. Furthermore, when ECs are treated with digoxin antibody for one (1) hour after induction with TNF-α, the antibody reduces the TNF-α induced expression of ICAM, VCAM and E-selectin, as shown, respectively, in **Figures 5A, 5B** and **6****.**

### Prophetic Biological Example #2:

Digoxin antibody is known to restore Na⁺/K⁺ ATPase activity in erythrocyte cell membranes. As demonstrated in Biological Example #1, digoxin antibody attenuates TNF-induced adhesion molecule expression, suggesting that altered Na⁺/K⁺ ATPase may contribute to endothelial cell activation in the inflammatory and/or immune response. To test this possibility, Na⁺/K⁺ ATPase activity is determined in various cells, preferably endothelial cells and leukocytes, of normal control (i.e. healthy) patients and patients exhibiting cytokine mediate conditions (e.g., sepsis, severe burn or trauma, HIV, cerebral malaria). The effect of digoxin antibody on Na⁺/K⁺ ATPase activity would be determined, and digoxin antibody would attenuate Na⁺/K⁺ ATPase inhibition in cells of patients exhibiting inflammatory-related or cytokine mediated conditions.

Animal models (e.g., rat, mouse, etc.) of sepsis are well known by those skilled in the art and any such model may be used in connection with present the invention. Rats (Wistar, male) are administered lipopolysaccharide ("LPS") (20 ng/kg) pr saline (control) through the femoral vein, and sacrificed at 2, 12, and 24 hours after LPS administration. Leukocytes, erythrocytes and endothelial cells are isolated from sacrificed LPS-treated and control rats and placed in a solution of HEPES buffer with ⁸⁶Rubidium (Rb⁺), a potassium analog, for three hours. Na⁺/K⁺ ATPase activity is determined by the uptake of the Rb. Na⁺/K⁺ ATPase activity is significantly decreased in LPS-treated rats compared to controls.

The effect of digoxin antibody on Na⁺/K⁺ ATPase activity is also evaluated. Digoxin antibody (DIGIBIND^{®} or DIGIFAB™ 0.1 mg digoxin binding capacity/kg) or normal saline placebo is administered to LPS-treated rats approximately 24 hours after LPS administration. Sets of rats are treated with digoxin antibody or saline placebo, and sacrificed at 2, 6, and 12 hours after therapeutic intervention. Endothelial cells, leukocytes and erythrocytes are isolated from digoxin antibody treated and control rats, and placed in solutions of HEPES buffer and Rb⁺. Na⁺/K⁺ ATPase activity is determined at baseline (before administration of digoxin antibody or placebo) and at 2, 6 and 12 hours after administration, by the uptake of Rb⁺ (expressed as nmol/hr/10⁶ cells). Mean Na⁺/K⁺ ATPase activity increases toward normal controls in both the digoxin antibody and placebo treated samples (reflecting the loss over time of the effect that LPS on sodium pump activity); however, for the placebo treated samples, the increase in activity is more gradual and modest. (See **Table VI** below).

**Table VI: Digoxin Antibody Effect on Sodium/Potassium ATPase Activity* in Endothelia from Septic Rats**

| Group | Baseline | 2 hours | 6 hours | 12 hours |
|---|---|---|---|---|
| Normal Control (non-septic) N=12 | 82 | 79.0 | 82 | 84 |
| DIGIFAB™ Treated Septic Samples (N=6) | 48 | 63 | 62 | 73 |
| Placebo Treated Septic Samples (N=6) | 44 | 52 | 54 | 60 |

| | | | | |
|---|---|---|---|---|
| *Activity expressed as ⁸⁶Rb⁺ uptake nmol/hr/10⁶ RBC | | | | |

### Prophetic Biological Example #3:

Sepsis is induced in C57/BL mice by the intravenous injection of 20 ng of LPS per animal plus 20 mgs D-galactosamine per animal. Inhibition of sepsis is measured as the prevention of mortality over a three day period. Digoxin antibody (0.01 mg digoxin binding capacity/kg) is administered intraperitoneally with normal saline one (1) hour before LPS induction. There was 100% mortality over the three day period for mice receiving placebo. Mice receiving digoxin antibody had 100% survival over the three day monitoring period.

### Prophetic Biological Example #4

A randomized, double-blind, placebo-controlled, multicenter trial is conducted. Patients with systemic inflammation (SIRS) and organ failure due to acute infection are enrolled and assigned to receive an intravenous bolus infusion of either placebo or digoxin immune Fab (ovine) (.04 mg digoxin binding capacity per kilogram of body weight, based on assumed serum TNF-α of 100 pg/ml), every six hours for a total duration of 96 hours. The prospectively defined primary end point is death from any cause and is assessed 28 days after the start of the infusion. Patients are monitored for adverse events, changes in vital signs, laboratory variables and the results of microbiologic cultures, and the development of neutralizing antibodies against digoxin immune Fab (ovine).

A total of 100 randomized patients are treated (48 in the placebo group and 52 in the digoxin immune Fab (ovine) group). The mortality rate is 30.8 percent in the placebo group and 24.7 percent in the digoxin immune Fab (ovine) group. On the basis of the prospectively defined primary analysis, treatment with digoxin immune Fab (ovine) is associated with a reduction in the risk of death of 6.1 percent (P=0.005).

## Claims

1. A pharmaceutical composition comprising an anti-digoxin antibody composition for use in the treatment of chronic and acute disorders, diseases, infections and injuries that are **characterized by** localized or systemic inflammatory responses and associated with or mediated or modulated by TNF.

2. The composition for use of claim 1, wherein said anti-digoxin antibody comprises digoxin immune Fab.

3. The composition for use of one of claims 1 to 2, wherein the inflammatory response is selected from sepsis, SiRS or hemorrhagic shock.

4. Use of an anti-digoxin antibody composition for the preparation of a medicament for the treatment of chronic and acute disorders, diseases, infections and injuries that are **characterized by** localized or systemic inflammatory responses and associated with or mediated or modulated by TNF.

5. The use of claim 4, wherein said anti-digoxin antibody comprises digoxin immune Fab.

6. The use of claim 4 or 5, wherein the inflammatory response is selected from sepsis, SiRS or hemorrhagic shock.

7. The composition for use according to one of claims 1 to 3 or the use according to one of claims 4 to 6, wherein the anti-digoxin antibody composition comprises an anti-digoxin antibody sufficient to provide between 0.001 mg and 500 mg digoxin binding capacity per kg patient weight.

## Patentansprüche

1. Pharmazeutische Zusammensetzung, die eine Anti-Digoxin-Antikörper-Zusammensetzung umfasst, zur Verwendung in der Behandlung chronischer und akuter Störungen, Krankheiten, Infektionen und Verletzungen, die durch lokalisierte oder systemische inflammatorische Reaktionen gekennzeichnet sind und mit TNF assoziiert sind oder durch TNF vermittelt oder moduliert werden.

2. Zusammensetzung zur Verwendung gemäß Anspruch 1, wobei der Anti-Digoxin-Antikörper Digoxin immune Fab umfasst.

3. Zusammensetzung zur Verwendung gemäß einem der Ansprüche 1 bis 2, wobei die inflammatorische Reaktion aus Sepsis, SiRS oder hämorrhagischem Schock ausgewählt ist.

4. Verwendung einer Anti-Digoxin-Antikörper-Zusammensetzung zur Herstellung eines Medikaments für die Behandlung chronischer und akuter Störungen, Krankheiten, Infektionen und Verletzungen, die durch lokalisierte oder systemische inflammatorische Reaktionen gekennzeichnet sind und mit TNF assoziiert sind oder durch TNF vermittelt oder moduliert werden.

5. Verwendung gemäß Anspruch 4, wobei der Anti-Digoxin-Antikörper Digoxin immune Fab umfasst.

6. Verwendung gemäß Anspruch 4 oder 5, wobei die inflammatorische Reaktion aus Sepsis, SiRS oder hämorrhagischem Schock ausgewählt ist.

7. Zusammensetzung zur Verwendung gemäß einem der Ansprüche 1 bis 3 oder Verwendung gemäß einem der Ansprüche 4 bis 6, wobei die Anti-Digoxin-Antikörper-Zusammensetzung einen Anti-Digoxin-Antikörper, ausreichend, um zwischen 0,001 mg und 500 mg Digoxin-Bindungskapazität pro kg Patientengewicht bereitzustellen, umfasst.

## Revendications

1. Composition pharmaceutique comprenant une composition d'anticorps anti-digoxine pour l'utilisation dans le traitement des troubles, des maladies, des infections et des blessures aigus ou chroniques, qui sont **caractérisés par** des réactions inflammatoires localisées ou systémiques et qui sont associés avec TNF ou sont provoqués par l'intermédiaire de ou modulés par TNF.

2. Composition pour l'utilisation selon la revendication 1, dans laquelle ledit anticorps anti-digoxine comprend la digoxine immune fab.

3. Composition pour l'utilisation selon l'une quelconque des revendications 1 à 2, la réaction inflammatoire étant choisie dans le groupe constitué de la septicémie, siRS ou un choc hémorragique.

4. Utilisation d'une composition d'anticorps anti-digoxine pour la préparation d'un médicament pour le traitement des troubles, des maladies, des infections et des blessures aigus ou chroniques, qui sont **caractérisés par** des réactions inflammatoires localisées ou systémiques et qui sont associés avec TNF ou sont provoqués par l'intermédiaire de ou modulés par TNF.

5. Utilisation selon la revendication 4, dans laquelle l'anticorps anti-digoxine comprend digoxine immune fab.

6. Utilisation selon la revendication 4 ou 5, la réaction inflammatoire étant choisie dans le groupe constitué de la septicémie, siRS ou un choc hémorragique.

7. Composition pour l'utilisation selon l'une quelconque des revendications 1 à 3 ou l'utilisation selon l'une quelconque des revendications 4 à 6, dans laquelle la composition d'anticorps anti-digoxine comprend un anticorps anti-digoxine, suffisant pour fournir une capacité de liaison pour digoxine comprise entre 0,001 mg et 500 mg par kg de poids du patient.
